(19) Europäisches Patentamt
European Patent Office
Office européen des brevets

(11) EP 3 231 791 B1

(12) **EUROPEAN PATENT SPECIFICATION**

(45) Date of publication and mention of the grant of the patent:
**15.09.2021 Bulletin 2021/37**

(21) Application number: **15867255.0**

(22) Date of filing: **27.11.2015**

(51) Int Cl.:
***C07C 309/69*** *(2006.01)* ***C07C 303/26*** *(2006.01)*
***C10G 75/02*** *(2006.01)* ***C23F 11/16*** *(2006.01)*
***C10L 1/24*** *(2006.01)*

(86) International application number:
**PCT/MX2015/050001**

(87) International publication number:
**WO 2016/093688 (16.06.2016 Gazette 2016/24)**

(54) **HYDROXYSULTAINE- AND SULFOBETAINE-BASED GEMINAL ZWITTERIONIC LIQUIDS, METHOD FOR OBTAINING SAME, AND USE THEREOF AS WETTABILITY MODIFIERS HAVING CORROSION INHIBITING PROPERTIES**

HYDROXYSULTAIN- UND SULFOBETAINBASIERTE ZWITTERIONISCHE GEMINALE FLÜSSIGKEITEN, VERFAHREN ZUR HERSTELLUNG DAVON UND VERWENDUNG DAVON ALS BENETZBARKEITSMODIFIKATOREN MIT KORROSIONSHEMMENDEN EIGENSCHAFTEN

LIQUIDES ZWITTERIONIQUES GÉMINAUX, PROCÉDÉ D'OBTENTION ET UTILISATION EN TANT QUE MODIFICATEURS DE LA MOUILLABILITÉ PRÉSENTANT DES PROPRIÉTÉS ANTI-CORROSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.12.2014 MX 2014015226**

(43) Date of publication of application:
**18.10.2017 Bulletin 2017/42**

(73) Proprietor: **Instituto Mexicano Del Petróleo**
**Del. Gustavo A. Madero,**
**México, D.F. 07730 (MX)**

(72) Inventors:
- **HERNÁNDEZ ALTAMIRANO, Raúl**
  **07730 Distrito Federal, México (MX)**
- **MENA CERVANTES, Violeta Yasmín**
  **07730 Distrito Federal, México (MX)**
- **ZAMUDIO RIVERA, Luis Silvestre**
  **07730 Distrito Federal, México (MX)**
- **FLORES SANDOVAL, César Andrés**
  **07730 Distrito Federal, México (MX)**
- **RAMÍREZ ESTRADA, Alejandro**
  **07340, Ciudad de México, México (MX)**
- **CISNEROS DEVORA, Rodolfo**
  **07730 Distrito Federal, México (MX)**
- **MARTÍNEZ MAGADAN, José Manuel**
  **07730 Distrito Federal, México (MX)**
- **OVIEDO ROA, Raúl**
  **07730 Distrito Federal, México (MX)**
- **RAMÍREZ PÉREZ, Jorge Francisco**
  **07730 Distrito Federal, México (MX)**

(74) Representative: **Scholz, Volker**
**Boehmert & Boehmert**
**Anwaltspartnerschaft mbB**
**Pettenkoferstrasse 22**
**80336 München (DE)**

(56) References cited:
**MX-A- 2009 013 704**
**MX-A- 2012 011 116**
**MX-A- 2012 011 116**
**MX-A- 2012 014 187**
**US-A- 4 216 097**
**US-A- 4 383 054**
**US-A1- 2011 138 683**

- **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 231 791 B1

## Description

### TECHNICAL FIELD OF THE INVENTION

The present invention is located in the multifunctional chemical products area applied to the oil industry for enhanced recovery processes, specifically in the obtaining and use of geminal zwitterionic liquids sulfobetaine- and hydroxysultaine-base with wettability modifying properties of carbonate rocks and heterogeneous lithology, in the presence of brines with a high content of divalent ions such as calcium, magnesium, barium and strontium, high temperature and high pressure.

The geminal zwitterionic liquids sulfobetaine- and hydroxysultaine-base also have the property of acting as corrosion inhibitors in crude oil production pipelines.

### BACKGROUND OF THE INVENTION

The geminal zwitterionic liquids (1) constitute a family characterized by having hydrocarbon chains (A), a bridge (B) and two polar groups of zwitterionic type (C).

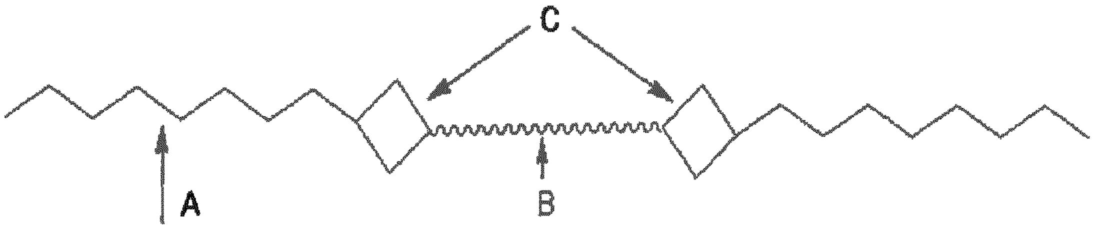


(1) General structure of a geminal zwitterionic liquid.

Geminal zwitterionic liquids are electrically neutral compounds, but have formal positive (2 cations) and negative (2 anions) charges on different atoms of the same molecule. They are molecules capable of adapting to different media and therefore can be designed to respond efficiently depending on the contaminants and the operating conditions where they are applied.

Below, there are some examples of chemical structures of zwitterionic liquids sulphonate-base, that are reported in the literature (2) *[*Chemistry Letters, 2004, 33 (12), 1594-1595; Latvian Journal of Chemistry, 2010, (1-4), 102-113; Monatshefte für Chemie, 2008, 139, 799-803].

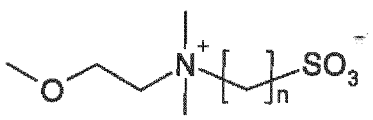

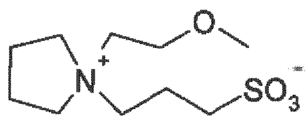

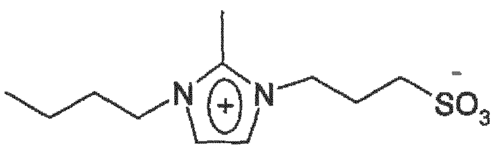

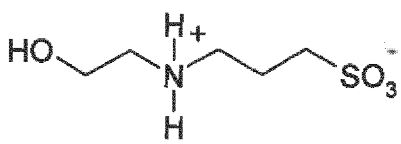

(2) Chemical structures of some zwitterionic liquids reported in the literature.

For the particular case of the increase in oil production, is have that, after primary and secondary recoveries, the oil reservoir still contains 50-80% of crude oil originally in place. This is due to that the primary and secondary recovery methods efficiency is limited by two factors:

- At pore scale, the crude oil can reach a residual saturation sufficiently low to be found in the form of discontinuous globules, trapped by capillary forces.

- At reservoir scale, there exist certain areas in which the injected fluid for the secondary recovery does not infiltrate, as a result of the low permeability of these zones.

The currently proposed enhanced oil recovery methods are aimed at the solution of the above declared problems and the use of wettability modifiers surfactant chemicals is one of the methods most widely used, within these surfactants there are cationic, anionic, non-ionic and zwitterionic or mixture of these chemicals.

A wettability modifier is defined as a surfactant that is able to change favorably the reservoir rock affinity. The wettability is a measure of the interaction among the existing phases in the reservoir, and it is a function of the interfacial chemistry of these phases and determines the tendency of a fluid to move forward or remain adhered to a solid surface in the presence of other immiscible fluids. The rock wettability can be modified naturally by the adsorption of polar compounds, the organic material deposits formation that were originally in oil or by different external agents. Wettability changes affect the capillary pressure, relative permeability, residual oil saturation and irreducible water saturation.

Despite of the continued progress in chemical wettability modifiers development, currently, there exist reservoir sites very difficult to handle, mainly because they are naturally fractured reservoirs, have low matrix permeability, heterogeneous lithology, high temperatures, above 90 °C, high salinity, greater than 60,000 ppm as sodium chloride, and a high content of divalent ions, mainly calcium and magnesium, more than 5,000 ppm.

As a result of the just mentioned above, the characterization of the reservoir type of rock, as well as the composition of adsorbed crude oil and its surrounding environment, it is of the utmost importance in designing new wettability modifiers and even to propose molecular structures: to be tolerant to brine salt saturated, mainly calcium and magnesium; having a good diffusion through the medium, generally composed of brine and oil; and having moieties with polar groups with affinity for the rock, in order to be able to favorably modify the wettability from oil-wet to water-wet.

Globally, there are a variety of specific cases of chemicals that have been successfully used to solve those problems, for example anionic surfactants such as sodium alkyl sulfonates or cationic surfactants like alkyl ammonium chlorides, but unfortunately their application is not universal, because of the conditions present in Mexican reservoirs are quite different from other countries, for this reason, the development of more versatile chemicals is of utmost importance, they have to be used in the most adverse known conditions, and they ought to be able to simultaneously solve the greatest number of problems, as for example the corrosion that is directly associated with the use of sea- or connate-water normally used as the solvent of the wettability modifier chemical with the purpose of minimize implementation costs when they are injected into the reservoir.

In order to increase the crude oil recovery factor, they have been developed wettability modifiers chemicals such as those cited below:

US. Pat. No. 5,042,580 (Oil Recovery Process for use in fractured reservoirs) protects an enhanced oil recovery process that involves the wettability modifier injection into the reservoir, consisting of a mixture of different types of surfactants just like alkyl sulfonate and chrome salts, derived from fatty carboxylic acids.

US. Pat. No. 4,509,951 (Enhanced recovery by imbibition process) protects an enhanced recovery process that involves the wettability modifier addition in the reservoir, consisting of a mixture of different types of products, within which are ammonium salts, hydroxides of alkalis metals, alkyl tripolyphosphates, and carbonates and bicarbonates of alkali metals.

US. Patent Application 2009/0023618 A1 (Method of Oil Recovery) protects an enhanced recovery process that involves the injecting to the reservoir of a Wettability modifier consisting of a mixture of different types of organophosphorus compounds.

US. Pat. No. 4,842,065 (Oil Recovery Process using a Wettability Modifying Cyclic Process) protects an enhanced recovery process that involves the injecting to the reservoir of a wettability modifier that consisting of a mixture of different types of ethoxylated alcohols.

US. Pat. No. 3,643,738 (Control of Wettability in Oil Recovery Process), protects a process that it allows the change of wettability through the use of mixtures of petroleum sulfonates.

US2011/138683 discloses geminal zwitterionic compositions for use as multifunctional corrosion inhibitors.

MX patent 318024 refers to a geminal zwitterionic liquid composition, amino acid-base with wettability modifying properties in enhanced oil recovery processes with structural formula:

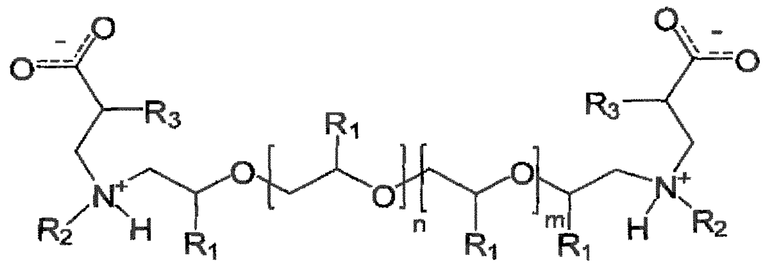

(3) Structural formula of geminal zwitterionic liquids of the MX 318024 patent.

It should be noted the zwitterionic liquids of that patent differ from the present invention since they are not of the sulfobetaine or hydroxysultaine-type.

In relation to the use as corrosion inhibitors with application in hydrocarbons production and transportation processes, the specialized literature mentions the main chemical families that have been used are: 1) 1-heteroalkyl-2-alkyl Imidazolines (Patent MX **254565,** Corrosion Inhibitory Composition for Ferrous Metals in Acid Media, MX Patent **260049,** Corrosion Inhibitory Composition and Hydrogen Ampoulement to Ferrous Metals in Basic Media) 2) Alkyl Amide Amines (Journal of Chemical Society of Mexico 2002, 46 , 4, 335-340, Carbon Steel Corrosion Control in Hydrogen Sulfide Environments by 1-(2-Hydroxyethyl) -2-Alkyl-Imidazolines and their corresponding Amide Precursors; Applied Surface Science 2006, 252, 6, 2139 -2152, Surface Analysis of Inhibitor Films Formed by Imidazolines and Amides on Mild Steel in an Acidic Environment), 3) Polyalkylene polyamines (US Patent 4,900,458**,** Polyalkylenepolyamines as Corrosion Inhibitors; US Patent 4,275,744**,** Derivatives of Polyalkylenepolyamines as Corrosion Inhibitors) 4) Acetylenic alcohols (US Patent 5084210, Corrosion inhibitors), 5) Diacetylenic alcohols (U.S. Patent 4,039,336**,** Diacetylenic Alcohol Corrosion inhibitors), 6) Quaternary Ammonium Salts (U.S. Patent 6,521,028**,** Low Hazard Corrosion Inhibitors and Cleaning Solutions Using Quaternary Ammonium Salts), 7) Bis-imidazolines (MX Patent **246603,** Multifunctional, Biodegradable and Low Toxicity Corrosion Inhibitors) and 8) Bis-Quaternary Ammonium Salts (U.S. Patent Application 2006/0013798, Bis-Quaternary Ammonium Salt Corrosion Inhibitors). Also, due to the impact of corrosion phenomenon in the oil industry when there are high salinities and divalent ions concentrations, at international level different institutions and companies have been working on the development of new chemical structures with improved properties, for example US Pat. No. 8,105,987 (Corrosion Inhibitors for an Aqueous Medium) and U.S. Patent No. 2011/0138683 (Gemini Surfactants, Process of Manufacture and Use as Multifunctional Corrosion Inhibitors).

It is important to remark that in none of the aforementioned references, is referred the use of geminal zwitterionic liquids, hidroxisultaine and sulfobetaine-base neither it is suggested their obtaining process, nor their use as wettability modifiers with corrosion inhibiting properties, which alter the rock wettability favorably in enhanced oil recovery processes a wide range of rocks such as limestone, dolomite, sandstone, quartz or heterogeneous lithologies, and that these zwitterionic liquids may be exposed to brines with high content of divalent ion as they are calcium, magnesium, barium and strontium (150,000ppm), temperatures up to 220 °C; pressures up to 300 kg/cm$^2$; and they prevent and control the corrosion in pipeline of crude oil production in enhanced oil recovery processes.

## BRIEF DESCRIPTION OF THE FIGURES OF THE INVENTION

In order to have a better understanding regarding the application of geminal zwitterionic liquids, sulphobetaine and hydroxysultaine-base, obtaining process and use as wettability modifiers with corrosion inhibiting properties of the present invention, next reference will be made to the accompanying figures:
The Figure 1 shows the production of oil in the Amott cell capillary for: a) system that only contains brine, b) a system containing brine and 300 ppm of geminal zwitterionic liquid, sulfobetaine-base described in Example 2.

The Figure 2 shows the drop forms: a) system that only contains brine, b) system containing brine and 300 ppm of geminal zwitterionic liquid, sulfobetaine-base described in Example 2.

## DETAILED DESCRIPTION OF THE INVENTION

The present invention relates to obtaining and use of geminal zwitterionic liquids, hidroxisultaine-and sulfobetaine-base, as wettability modifiers of rocks such as limestone, dolomite, sandstone, quartz or heterogeneous lithology in the presence of crude oil and brine water with high content of divalent ions as calcium, magnesium, barium, and strontium, high temperature and pressure in enhanced oil recovery processes.

The geminal zwitterionic liquids type hydroxysultaine and sulfobetaine of the present invention have also the property of acting as corrosion inhibitors, in extraction, production, transport, and storage operations, properly preventing

and controlling the generalized and localized corrosion problems. An additional advantage presented by the zwitterionic liquids of the current invention is that due to its molecular structure, they may be soluble in brine, crude oil or organic solvents such as toluene, xylenes, chloroform and alcohols.

The geminal zwitterionic liquids of the present invention constitute a family characterized by having hydrocarbon chains, a polyether type bridge and two zwitterionic type polar groups which may be hydroxysultain or sulfobetaine-type.

The geminal zwitterionic liquids, hidroxisultaine- and sulfobetaine-base of the present invention have the following structural formula:

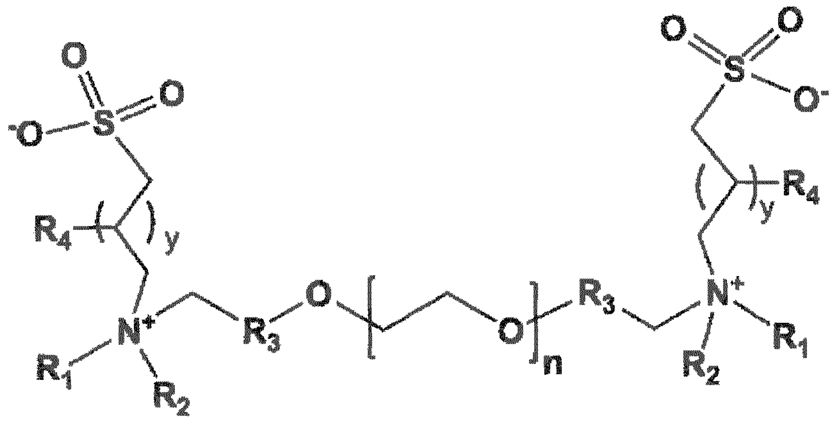

Where:

**R1** = is a radical represented by alkyl or alkenyl chain, linear or branched, preferably of 1 to 30 carbon atoms; or an cyclo alkyl or aryl group, preferably of 5 to 12 carbon atoms;

**R2** = is a radical represented by alkyl or alkenyl chain, linear or branched, preferably of 1 to 30 carbon atoms; or an cyclo alkyl or aryl group, preferably of 5 to 12 carbon atoms;

**R3** = -CH2-, -CH2-CH(OH)-;

y = has values of values from 0 to 1;

When y is equal to 1:

**R4** = is a radical represented by -OH

**n** has values of values from 1 up to 500.

For the development of the present invention a procedure was followed consisting of the following stages: 1) molecular design through computational chemistry, 2) synthesis and characterization of geminal zwitterionic liquids, sulfobetaine and hidroxisultaine-base 3) experimental evaluation of wettability modifier and corrosion inhibitory properties.

An additional advantage presented by the geminal zwitterionic liquids sulfobetaine and hidroxisultaine-base derived from their molecular structure is that they are soluble in water, brine or oil.

**1) Molecular Design by means of Computational Chemistry.** For clarification purposes, and before going into details, it is worth mentioning that the current trend, involving the development of new chemical products with industrial application, is as the first stage the molecular design by means of computational chemistry, of moieties that will have the potential ability to solve the problems of concern. This molecular design is intended to systematically direct the efforts aimed to the synthesis of new molecules with enhanced properties.

The first thing to determine within molecular design for problems related with wettability modification and corrosion inhibition is:

**i)** The interaction forces magnitude between the characteristic rock surface of a given reservoir and the polar molecules of high molecular weight in the crude oil, since these data constitute the reference energy that must be overcome by the appropriate insertion of a wettability modifier agent.

**ii)** It also requires a deep understanding of the wettability modification molecular mechanisms with the objective of suggest a surfactant with the most suitable molecular structure.

**iii)** In addition, in connection to the viscosity reduction application is required to determine the nature and magnitude of the intermolecular forces that generate oil high viscosity with a high content of polar compounds of large molecular weight such as asphaltenes and resins. These data provide the reference energy that must be overcome by insertion of an appropriate viscosity reducer agent.

**iv)** Finally, optimal structural parameters that it should include a wettability modifier and viscosity reducer molecule are selected, this from a technical-economic standpoint in order to propose an initial molecular structure to which it calculates the interaction energy with rock surface to determine whether this interaction will be greater or lesser in energy with respect to the polar organic compounds of high molecular weight, which are required to be removed from the surface in such a way to increase the oil recovery factor in the reservoir. At this point, the process can be iterative to find a molecule that actually possesses a bigger energy of interaction with the rock than the adsorbed polar compounds of the oil.

Specialized literature mention that the effectiveness of wettability change in rock depends on the ionic nature of the involved surfactant (Colloids Surf. A: Physicochem. Eng. Aspects 1998, 137, 117-129. Austad, T.; Matre, B.; Milter, J.; Saevareid, A.; Oyno, L. Chemical flooding of oil reservoirs 8. Spontaneous oil expulsion from oil- and water-wet low permeable chalk material by imbibition of aqueous surfactant solutions; J. Pet. Sci. Eng. 2000, 28, 123-143. Standnes, D. C.; Austad, T. Wettability alteration in chalk: 2. Mechanism for wettability alteration from oil-wet to water-wet using surfactants) and that in oil-wet limestone cores, cationic surfactants have better perform than anionic. It has been proposed that in the case of cationic surfactants, the mechanism through which the wettability is modify in an oil-wet rock, is the formation of ion pairs between the surfactant cationic head and the oil acidic components adsorbed on the carbonate rock surface.

This ion pair formation could remove the layer of crude oil components adsorbed on the rock surface, thus exposing the calcium carbonate rock surface and this becomes water-wet. In contrast, anionic surfactants would form a monolayer on the rock surface through hydrophobic interaction mechanism between the tails of surfactant molecules and crude oil components adsorbed on the rock surface. In this way, the rock would be covered by a surfactant molecules layer which would not have the ability of self-assembly on the surface, allowing the rock wettability tends to be preferentially water-wet.

In the case of the present invention, geminal zwitterionic liquids, hidroxisultaine- and sulfobetaine-base have the ability to alter the wettability of carbonate rocks from oil-wet to water-wet through the two mechanisms stated above in parallel form, and hence presenting more efficiency than the cationic or anionic surfactants traditionally have been used.

In addition, the duality in the charge they have in the structure would allow interact effectively with different types of rock and oils, as they may form ionic pairs with species both positive and negative charge.

Just as it has been addressed in the Background of the Invention, zwitterionic surfactants present significant advantages compared to cationic or anionic surfactants because as zwitterions have both positive and negative charges in its structure, which increases the possibility to interact with rock surfaces with both positively and negatively charged, in contrast with one only type of charge surfactants. In many oil reservoirs at international level, there are present the cases of heterogeneous lithology that hinder the enhanced oil recovery process by wettability change, in these cases is required use wettability modifiers that they are effective in contact with different types of rock such as limestone, dolomite, sandstone, quartz or heterogeneous lithology, even in the presence of brine with high content of divalent ions such as calcium, magnesium, barium and strontium, high temperature and pressure in enhanced oil recovery processes in order to increase oil production.

From the previous data, the generation of branched geminal zwitterionic liquids presents advantages over conventional surfactants, for its application as wettability modifiers in different types of lithology under high salinity and temperature conditions.

In order to prove the above declared premises, theoretical calculations were performed simulating the adsorption process of polar molecules of high molecular weight on the rock surface originally water-wet for determine the adsorption energy that must overcome the wettability modifier molecule; in the same way it was simulated the branched geminal zwitterionic liquid sulfobetaine-type adsorption process, corresponding to the present invention, on the same rock which is originally water-wet; for the purpose of determining the adsorption energies and compare it to establish whether from the theoretical point of view the asphaltene desorption process would be thermodynamically favorable entering a modifier of the wettability of the present invention. The results are described in the following examples:

**Example 1**

**Asphaltene-calcite interaction.** Through computational chemistry and using quantum methods that employ the Density Functional Theory and the LDA-VWN functional, was optimized in a water solvated medium (dielectric constant 78.54), the geometry of the molecular structure, model of asphaltene which represents the characteristics of a heavy oil from the Marine Region in Mexico 1 (Adapted based on the doctoral thesis by Tomás Eduardo Chávez Miyauchi; Design of new multifunctional compounds applied to the development of flow improvers for heavy and extra-heavy crude oil; Instituto Mexicano del Petróleo 2013, Page 126) the geometry of the calcite surface ($CaCO_3$) **2** and the geometry of the adsorption product **3** generated from the interaction of the molecular structure, model of asphaltene **1** with the calcite surface ($CaCO_3$) **2** (**4**) and the energetics results obtained for the adsorption process of the molecular structure, model of asphaltene **1** on the calcite surface ($CaCO_3$) **2** (**5**) are shown in Table **1.**

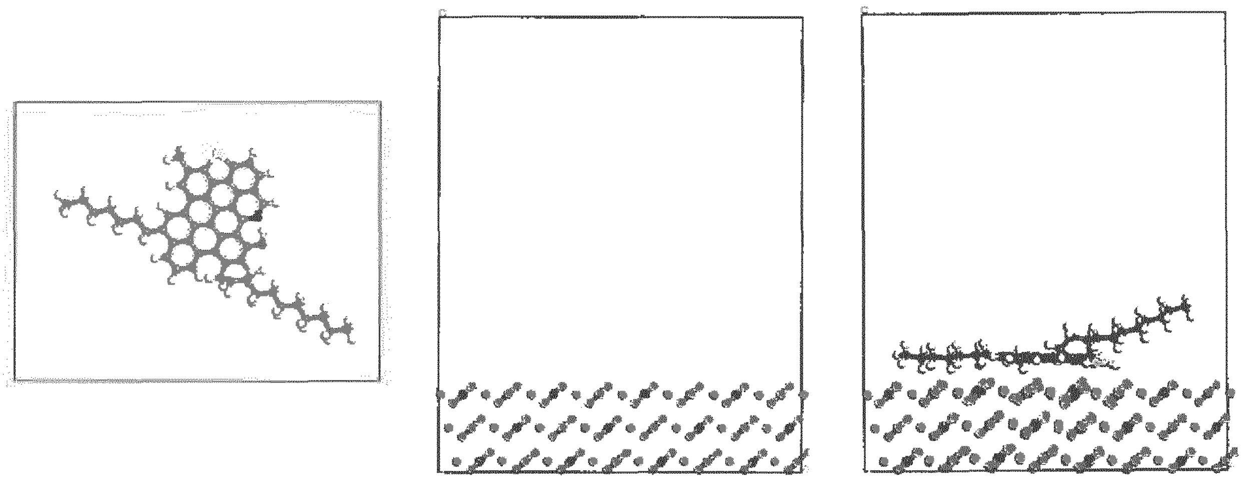

1 2 3

**(4)**

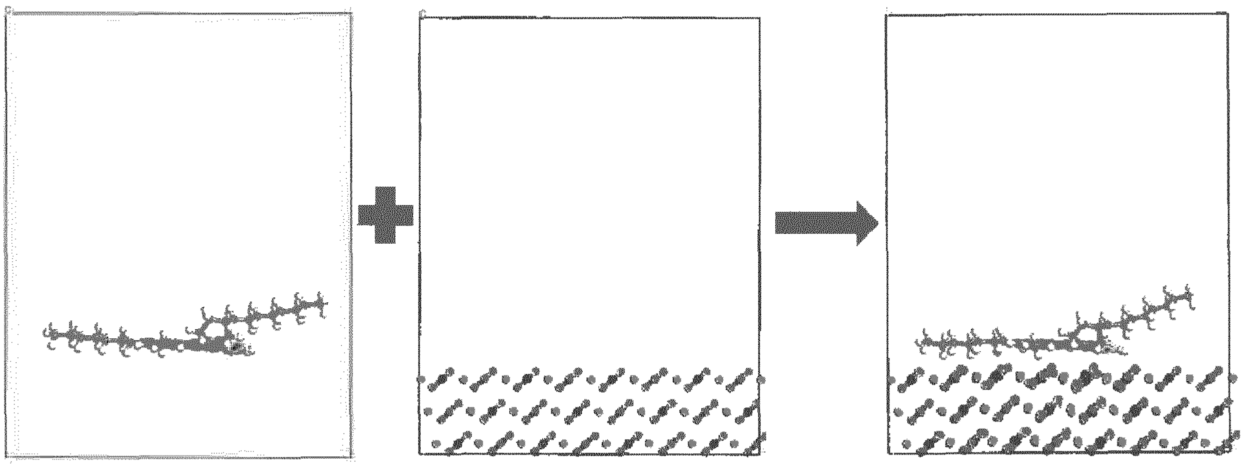

1 2 3

**(5)**

**Tabla 1.** Energetics results obtained for the adsorption process of a molecular structure of asphaltene 1 on the calcite surface ($CaCO_3$) **2** obtained with the Density Functional Theory and the LDA-VWN functional in a solvated medium by water.

| Compound or Complex | Density Functional Theory and the LDA-VWN functional. | |
|---|---|---|
| | Total Energy (kcal/mol) | Interaction Energy (kcal/mol) |
| 1 | -1,605,263.02 | |
| 2 | -70,629,863.94 | -211.83 |
| 3 | -72,235,338.79 | |

According to the Table 1 results, it is observed the absorption of the asphaltene on the calcite surface would be a thermodynamically favorable process, due to the negative sign, with -211.83 kcal/mol of magnitude. This example simulates the obtaining of a carbonated rock, oil-wettable, process that is experimentally attributed to the adsorption of polar compounds of petroleum on rock for hundreds and thousands of years; and is the energy that must overcome the wettability modifier injected into the reservoir in a process of enhanced recovery.

**Example 2**

Through computational chemistry and using quantum methods that employ the Density Functional Theory and the LDA-VWN functional, was optimized in a water solvated medium (dielectric constant 78.54), the geometry of the molecular structure, model of asphaltene which represents the characteristics of a heavy oil from the Marine Region in Mexico (Adapted based on the doctoral thesis by Tomás Eduardo Chávez Miyauchi; Design of new multifunctional compounds applied to the development of flow improvers for heavy and extra-heavy crude oil; Instituto Mexicano del Petróleo 2013, Page 126) 1 the geometry of the dolomite surface ($CaMg(CO_3)_2$) **4** and the geometry of the adsorption product **5** generated from the interaction of the molecular structure, model of asphaltene **1** with the dolomite surface ($CaMg(CO_3)_2$) **4** (**6**) and the obtained energetics results for adsorption process of the asphaltene **1** molecular structure model on the dolomite surface (CaMg $(CO_3)_2$) **4**(**7**) are shown in Table **2**.

In accordance with the Table 2 results, it is observed that the asphaltene adsorption over the dolomite surface would be a thermodynamically favorable process with a magnitude of 194.08 kcal/mol.

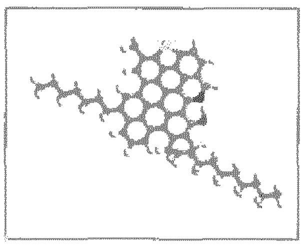

1

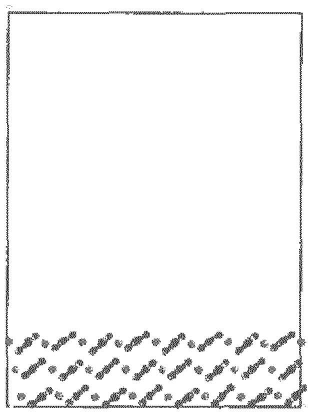

4

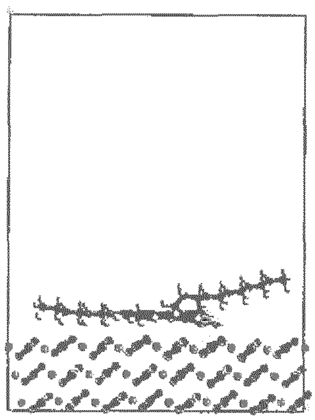

5

(6)

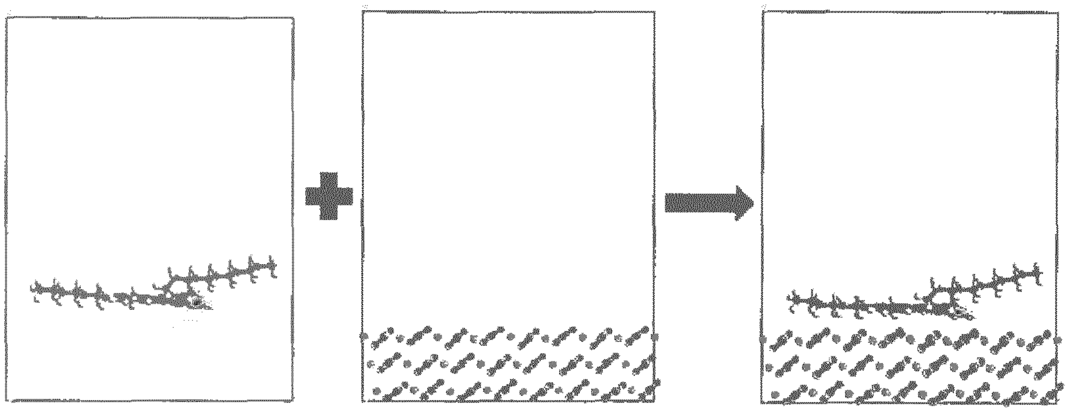

1 4 5

(7)

**Table** 2. The obtained energetics results for the adsorption process of the asphaltene 1 molecular structure model on the dolomite surface (CaMg $(CO_3)_2$) **4,** determined with the Density Functional Theory and the LDA-VWN functional in water solvation medium.

| Compound or Complex | Density Functional Theory and the LDA-VWN functional. | |
|---|---|---|
| | Total Energy (kcal/mol) | Interaction Energy (kcal/mol) |
| **1** | -1,605,263.16 | |
| **4** | -52,685,361.02 | -194.08 |
| **5** | -54,290,818.26 | |

**Example 3**

By way of computational chemistry by using quantum methods that employ the Density Functional Theory and the LDA-VWN functional, were optimized in a water solvation medium (dielectric constant 78.54): the geometry of the geminal zwitterionic liquid molecular structure, sulfobetaine-base of the present invention **6;** the geometry of the calcite surface ($CaCO_3$) **2;** and the geometry of the adsorption product **7** generated from the interaction of the molecular structure of the zwitterionic liquid **6** with the calcite surface ($CaCO_3$) 2(9); as are shown in **Table 3.**

According to the **Table 3** results, it is observed the absorption of the geminal zwitterionic liquid, sulfobetaine-base of the present invention on the calcite surface ($CaCO_3$) would be a thermodynamically favorable process, due to the negative sign, with -296.04 kcal/mol of magnitude. Moreover, comparing the latter result with the one presented in **Table 1** corresponding to the asphaltene-calcite interaction, hence, it is observed that the zwitterionic liquid-calcite interaction is favored by -80.21 kcal/mol, so it is verified from the theoretical view point that zwitterionic liquids have potential to properly function as wettability modifiers for rocks with a high tendency to be oil wettable such as limestone rocks ($CaCO_3$).

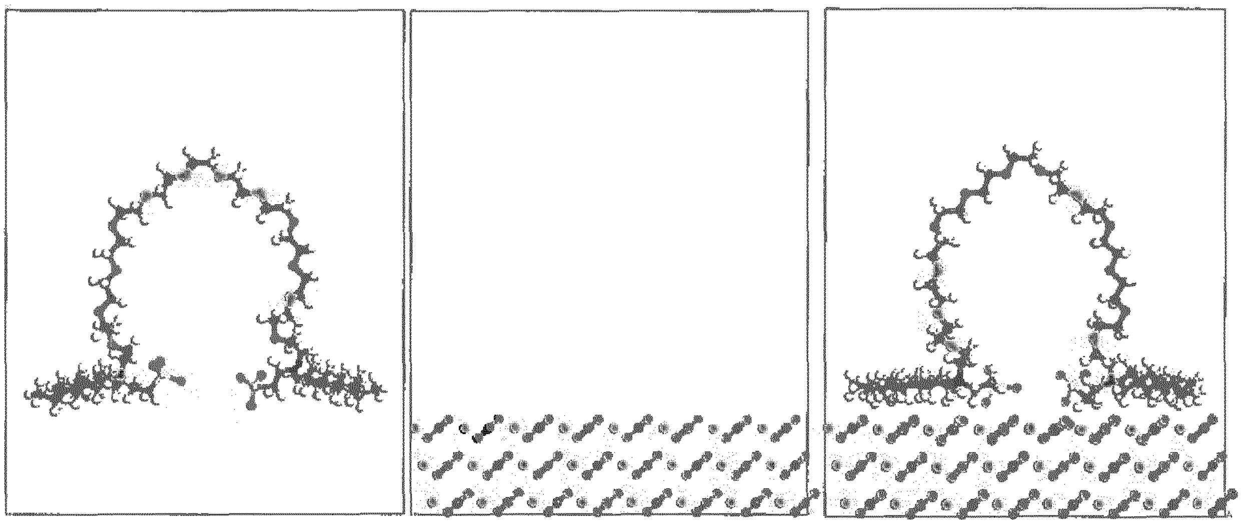

6 2 7

(8)

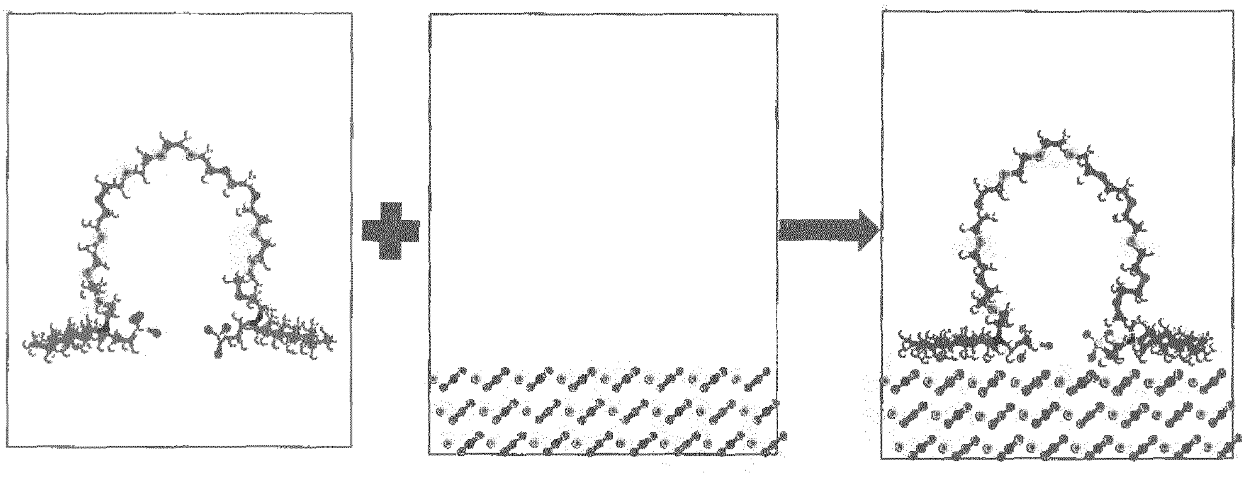

6 2 7

(9)

Table 3 Energetics results obtained for the adsorption process of a molecular structure of a geminal zwitterionic liquid, sulfobetaine-base **6** on the calcite surface ($CaCO_3$) **4** obtained with the Density Functional Theory and the LDA-VWN functional in an aqueous medium.

| Compound or Complex | Density Functional Theory and the LDA-VWN functional. | |
|---|---|---|
| | Total Energy (kcal/mol) | Interaction Energy (kcal/mol) |
| **6** | -2,926,840.81 | -292.04 |
| **2** | -70,629,863.72 | |
| **7** | -73,556,996.58 | |

**Example 4**

Through computational chemistry and using quantum methods that employ the Density Functional Theory and the LDA-VWN functional, was optimized in a water solvated medium (dielectric constant 78.54), the geometry of the molecular structure of a geminal zwitterionic liquid, sulfobetaine-base of the present invention **6,** the geometry of the dolomite surface ($CaMg(CO_3)_2$) **4(10)** and the energetics results obtained for the adsorption process of the molecular structure of the zwitterionic liquid 6 on the dolomite surface ($CaMg(CO_3)_2$) **4**(**11**), are shown in Table 4.

According to the Table 4 results, it is observed the absorption of the geminal zwitterionic liquid, sulfobetaine-base of the present invention on the dolomite surface ($CaMg(CO_3)_2$) would be a thermodynamically favorable process, due to the negative sign, with -261.64 kcal/mol of magnitude. Moreover, when comparing the latter result with the one presented in Table 2 corresponding to the asphaltene-dolomite interaction, it is observed that the zwitterionic liquid-dolomite interaction is favored by -67.52 kcal/mol, so it is verified from the theoretical view point that zwitterionic liquids possess the potential to function properly as wettability modifiers for rocks with a high tendency to be wettable by oil such as dolomite rocks ($CaMg(CO_3)_2$).

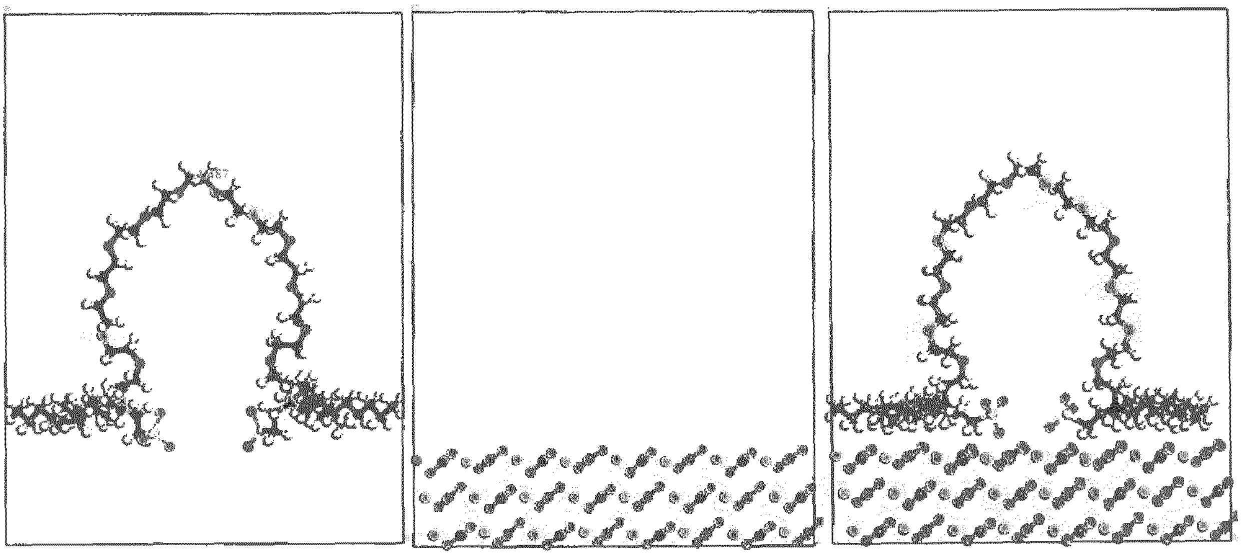

6 4 8

(10)

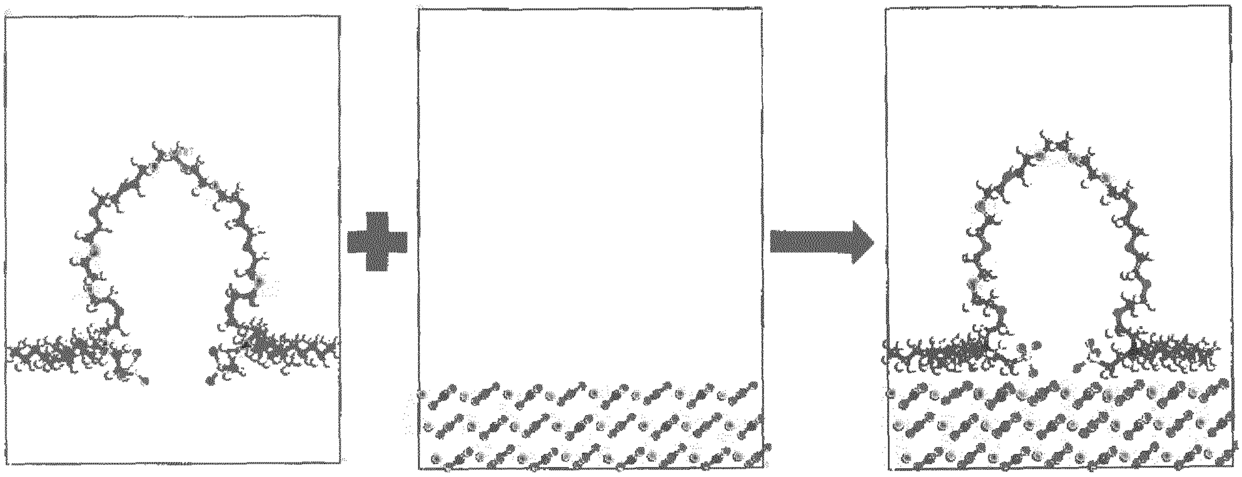

6 4 8

(11)

Table 4. Energetics results obtained for the adsorption process of a molecular structure of a geminal zwitterionic liquid, sulfobetaine-base **6** on the dolomite surface ($CaMg(CO_3)_2$) **4** obtained with the Density Functional Theory and the LDA-VWN functional in an aqueous medium.

| Compound or Complex | Density Functional Theory and the LDA-VWN functional | |
|---|---|---|
| | Total Energy (kcal/mol) | Interaction Energy (kcal/mol) |
| **6** | -2,926,839.73 | |
| **4** | -52,685,361.23 | -261.64 |
| **8** | -55,612,462.60 | |

**2) Synthesis and characterization of geminal zwitterionic liquids based on sulfobetaine and hydroxysultaine.** Once the molecular design stage has been completed, the zwitterionic liquids selected are synthesized at the laboratory level and subsequently evaluated to verify the design data.

Thus, the performance of the zwitterionic liquids of the present invention was evaluated experimentally as wettability modifiers in carbonated rocks and light- and heavy-crude oil, under high salinity and high temperature conditions and as corrosion inhibitors, under characteristic conditions of crude oil production pipeline.

The geminal zwitterionic liquids based on sulfobetaine and hydroxysultaine, object of the present invention are prepared according to the synthesis routes shown in the scheme (12) and comprise the following:

For **Route 1:** The first stage consists of reacting polyethylene glycols of formula I, derivatives of ethylene oxide with two hydroxyl groups, one at the end and the other at the beginning of the polymer chain, and whose molecular weight is in the range of 100 to 20,000 gr/mol; with benzenesulfonyl chloride or tosyl chloride; wherein the reaction is carried out with a molecular ratio of 1:1 to 1:4 between the polyethylene glycols of formula I and the benzenesulfonyl chloride or tosyl, preferably from 1:1.5 to 1:2.8; with an alkaline base of sodium, potassium or cesium, preferably sodium hydroxide; using as solvent water, tetrahydrofuran, chloroform or acetonitrile or mixtures of these; in a reaction time of 1 to 12 hours, preferably from 3 to 8 hours; and a temperature of 0 to 25 ° C, preferably from 5 to 20 ° C; to form the compounds of formula III.

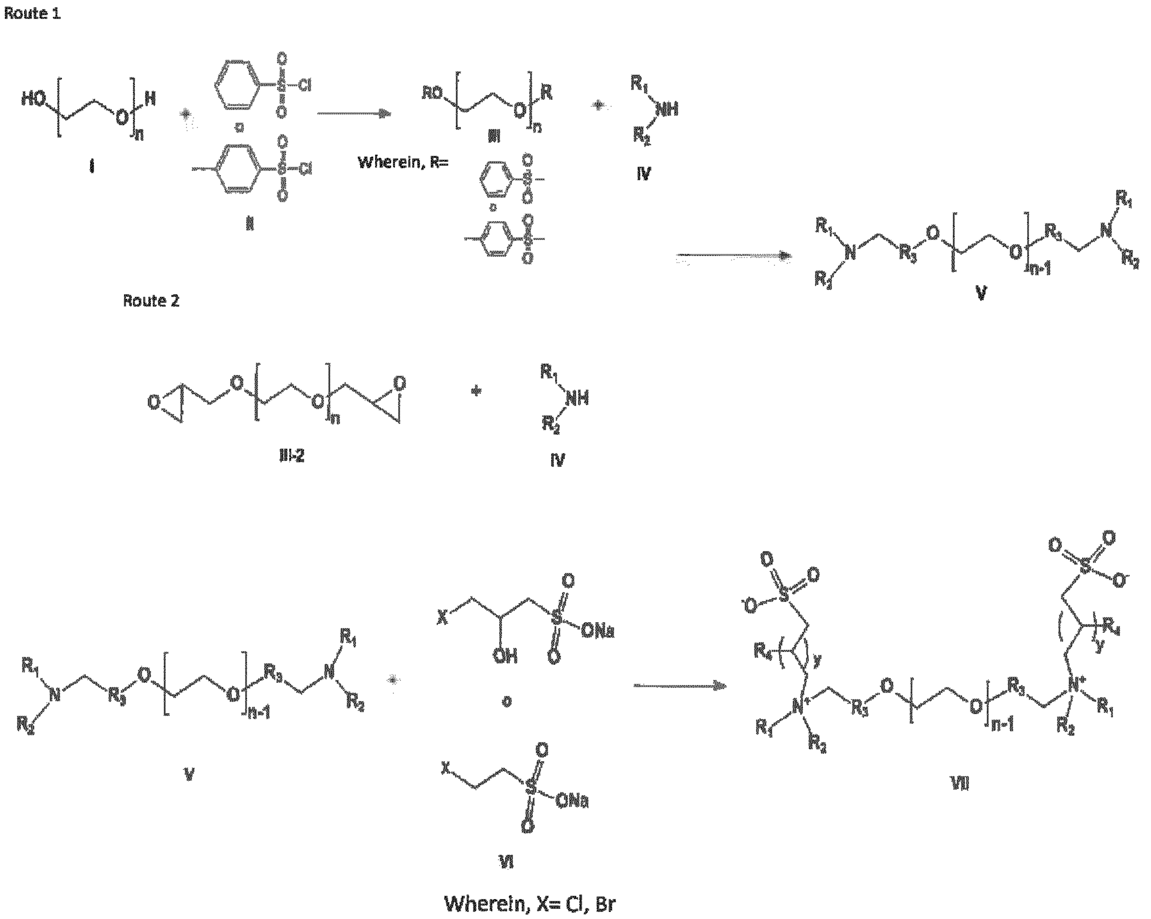


(12)

The second step consists in reacting the compounds of formula III through a nucleophilic substitution with amines of formula IV whose R1 and R2 can be alkyl or alkenyl, linear or branched chains, preferably from 1 to 30 carbon atoms; or cycloalkyl or aryl groups, preferably from 5 to 12 carbon atoms and wherein R2 can also be hydrogen and wherein the reaction is carried out in a molar reaction between the compounds of formula III and IV of 1:1.5 to 1:4, preferably from 1:1.8 to 1:2.6; in the presence of solvents such as acetonitrile, chloroform, dimethylformamide, dimethylsulfoxide, acetone or short chain alcohols; in a reaction time of 6 to 60 hours, preferably 36 hours; and at a temperature of 50 to 150 ° C; for obtaining the tertiary amines of formula V.

The third step consists in the reaction between the compounds of formula V with 2-bromo ethane sodium sulfonate of formula VI or sodium 3-chloro-2-hydroxypropane sodium sulfonate of formula VII, in a molar ratio of 1:1.5 to 1:4, preferably from 1:1.8 to 1:2.6; the reaction is carried out in the presence of solvents such as water or short-chain alcohols, preferably water; the reaction time, the temperature and the pressure depend on the structure of the compounds of formula V; generally the reaction time varies from 6 to 72 hours, preferably from 12 to 48 hours, the temperature from 40 to 180 °C, preferably from 80 to 130 °C, and the pressure is generally atmospheric, to obtain compounds of formul a VIII, geminal zwitterionic liquids, sulfobetaine- and hydroxysultaine-base.

For **Route 2:** The first step consists of reacting polyethylene glycol diglycidyl ether of formula I, with two epoxides groups, one at the end and the other at the beginning of the polymer chain, and whose molecular weight is in the range of 100 to 22,000 gr/mol with amines of formula IV whose R1 and R2 can be alkyl or alkenyl chains, linear or branched, preferably from 1 to 30 carbon atoms; or alkyl cycle or aryl groups, preferably from 5 to 12 carbon atoms and wherein R2 can also be hydrogen and wherein the reaction is carried out in a molar ratio between compounds of formula III and IV of 1:1.2 to 1:4, preferably from 1:1.8 to 1:2.6; without presence or with solvents such as water or short chain alcohols; in a reaction time of 4 to 15 hours, preferably 8 hours; and at a temperature of 50 to 150 ° C; to obtain the amino alcohols of formula V.

The second step consists of the reaction between the compounds of formula V with 2-bromine ethane sodium sulfonate of formula VI or with 3-chloro-2-hydroxypropane sodium sulfonate of formula VII, in a molar ratio of 1:1.5 at

1:4 preferably from 1:1.8 to 1:2.6; the reaction is carried out in the presence of solvents such as water or short-chain alcohols, preferably water; the reaction time, the temperature and the pressure depend on the structure of the compounds of formula V; generally the reaction time varies from 6 to 72 hours, preferably from 12 to 48 hours, the temperature from 40 to 180 °C, preferably from 80 to 130 ° C, and the pressure is generally atmospheric, to obtain the formula VIII, geminal zwitterionic liquids, sulfobetaine- and hydroxysultaine-base.

Next, some practical examples of obtaining branched gemmial zwitterionic liquids through above-mentioned reaction schemes to have a better understanding of the present invention, without this limiting its scope.

## Example 5

**Preparation** of **geminal zwitterionic liquid, sulfobetaine-base (product 1). First stage.** Into a 50 ml round flask containing 5.0 g of an aqueous solution of 17% weight sodium hydroxide (0.85 gr), 4.24 gr of polyethylene glycol whose molecular weight average in number is 600 gr/mol, the mixture was stirred for 20 minutes, subsequently, was added slowly at room temperature (20 °C) and atmospheric pressure, 7.37 gr of a solution of tosyl chloride at 40% weight (2.95 g) in tetrahydrofuran, maintaining throughout the entire addition a temperature below 25 ° C. Once the addition was complete, the reaction mixture was stirred for one hour at room temperature and atmospheric pressure; After the reaction mixture, the organic face was extracted and the solvent was evaporated at reduced pressure, obtaining 6.27 gr of product PE-TS (Diasylated polyether) as a transparent yellow viscous liquid.

As a **second stage.** Into a 50 ml round flask equipped with a refrigerant, magnetic stirrer and thermometer, 1.85 gr of dioctylamine, 6.27 g of PE-TS product, 4.77 g of potassium carbonate and 18 g of acetonitrile were placed. The reaction mixture was heated to reflux temperature and stirred vigorously for 8 hours.

At the end of the reaction time, an extraction of the organic phase was carried out and evaporated under reduced pressure to obtain 6.18 g of bis-N, N-dioctyl-N-polyether product as a viscous amber colored liquid.

As a **third stage.** In a 100 ml round flask equipped with a refrigerant, magnetic stirrer and thermometer, 6.18 gr of bis-N, N-dioctyl-N-polyether were placed and a solution of 2.77 gr of 2-bromine ethane sodium sulfonate in 50 gr of water. The reaction mixture was subjected to vigorous stirring, atmospheric pressure and reflux temperature for 48 hours.

After the reaction time had ended, the aqueous phase was separated and evaporated under reduced pressure. The dried product was washed with chloroform to filter out the salts present. The organic fraction was evaporated under reduced pressure to obtain 6.84 gr of the zwitterionic liquid as a viscous amber liquid called bis-N, N-dioctyl-N-polyether sulfobetaine.

The spectroscopic characteristics of product 1 are the following:

Representative bands of IR ($cm^{-1}$): 2922, 2853, 1641, 1464, 1221 y 1098.

Chemical shifts representative of NMR of $^1H$ ($CDCl_3$), 200MHz, δ(ppm): 0.86, 1.28, 1.75, 3.05, 3.27, 3.61 y 3.94 ppm.

Chemical shifts representative of NMR of $^{13}C$ ($CDCl_3$), 50 MHz, δ(ppm): 13.9, 22.3, 31.1, 52.1, 53.3, 65.4 y 70.4 ppm.

## Example 6

**Preparation of the geminal zwitterionic liquid, hydroxysultaine-base (Product** 2). The product bis-N, N-dioctyl-N-polyether was used, obtained in step 2 of example 7. In a 100 ml round flask equipped with a stirrer, magnetic stirrer and thermometer, 2 g of the bis-N, N-dioctyl-N-polyether and a solution of 0.86 gr of 3-chloro-2-hydroxy-propane sodium sulfonate in 15 g of water was added. The reaction mixture at atmospheric pressure and at reflux temperature was stirred vigorously for 72 hours.

After completion of the reaction time, the aqueous phase was separated and evaporated under reduced pressure. The dried product was washed with chloroform to filter out the salts present. The organic fraction was evaporated under reduced pressure to obtain 1.4 gr of the geminal zwitterionic liquid as a viscous amber liquid called bis-N, N-dioctyl-N-polyether hydroxysultaine. The spectroscopic characteristics of product 2 are the following:

Representative bands of IR ($cm^{-1}$): 3423, 2922, 2853, 1647, 1465, 1222 y 1098.

Chemical shifts representative of NMR of $^1H$ (CDCl3), 200MHz, δ(ppm): 0.83, 1.24, 1.26, 1.67, 2.99, 3.03, 3.20, 3.25,3.58 y 3.85 ppm.

Chemical shifts representative of NMR of $^{13}C$ (CDCl3), 50 MHz, δ(ppm): 13.8, 22.3, 31.0, 52.1, 53.2, 65.5 y 70.3 ppm.

**Example 7**

**Preparation of geminal zwitterionic liquid, sulfobetaine-base (Product 3). First stage.** In a 50 ml round flask containing 10 g of polyethylene glycol diglycidyl ether whose number average molecular weight is 526 gr/mol was added 3.5 g of dioctylamine, the mixture was stirred for one hour at a temperature of 95 ° C to obtain 13.5 g of the bis-N, N-dioctyl-N-hydroxypolieter product as a transparent yellow viscous liquid.

Two grams of bis-N, N-dioctyl-N-hydroxypolieter were placed in a 100 ml round flask equipped with a condenser, magnetic stirrer and thermometer and a solution of 0.86 gr of 2-bromine ethane sodium sulfonate in 15 gr of water. The reaction mixture at atmospheric pressure and reflux temperature, it was stirred vigorously for 72 hours.

After completion of the reaction time, the aqueous phase was separated and evaporated under reduced pressure. The dried product was washed with chloroform to filter out the salts present. The organic fraction was evaporated under reduced pressure to obtain 1.4 gr of the geminal zwitterionic liquid as a viscous amber liquid called bis-N, N-dioctyl-N-hydroxy polyether sulfobetaine. The spectroscopic characteristics of product 3 are as follows:

Representative bands of IR ($cm^{-1}$): 3423, 2922, 2853, 1647, 1465, 1222 y 1098.

Chemical shifts representative of NMR of $^1H$ (CDCl3), 200MHz, $\delta$(ppm): 0.83, 1.24, 1.26, 1.67, 2.99, 3.03, 3.20, 3.25,3.58 y 3.85 ppm.

Chemical shifts representative of NMR of $^{13}C$ (CDCl3), 50 MHz, $\delta$(ppm): 13.8, 22.3, 31.0, 52.1, 53.2, 65.5 y 70.3 ppm.

**3) Performance tests of branched geminal zwitterionic liquids as wettability modifiers and corrosion inhibitors.** For the evaluation of the wettability modifying properties of limestone, dolomite, sandstone, quartz rocks or heterogeneous lithology, in the presence of brines with a high content of divalent ions such as calcium, magnesium, barium and strontium, the recovery factor was determined by the process of spontaneous imbibition in limestone nuclei in Amott cells, in order to evaluate the efficiency of the wettability modifiers of the present invention with respect to a reference system without the presence of a chemical. In the evaluations, Bedford limestone nuclei composed mainly of calcium carbonate were selected, for being a rock which presents strong adsorption of organic polar compounds of the oil and therefore constitute the most severe case for a wettability modifier, which would guarantee that this type of chemical agents would work properly in another rock type with less tendency to be oil-wet. The test method is described below.

**Determination of the recovery factor by spontaneous imbibition process in limestone nuclei in Amott cells.** The test method consists of measuring the amount of crude oil recovered from carbonated rock cores initially saturated with oil due to spontaneous imbibition processes by water; in Amott cells at constant temperature and atmospheric pressure.

**Required elements for the test:**

- Amott cells.
- Recirculator of controlled temperature.
- Limestone cores of 2.81 cm in diameter and 7 cm long, with known permeabilities and porosities.
- Photographic camera.
- Crude oil.
- High salinity brine.
- Analytical balance.
- Extraction glass equipment SOXHLET.
- Glass volumetric material.
- Convection stove.

**Test procedure:**

1) They are subjected to extraction processes of hydrocarbons with different organic solvents in a SOXHLET system to the carbonated rock nuclei (dolomite or limestone). The extraction processes are carried out sequentially and at reflux; using as solvents: a) xylene, b) chloroform, c) methanol, d) xylene, e) chloroform, f) methanol and g) xylene.

The duration of each extraction stage is one day and the processing time is 7 days.

2) Determine the absolute helium permeability of the cores, as well as their effective porosity.

3) Dry the cores in a stove at a temperature of 100 °C and record the weight.

4) To put in contact, the nuclei with the dead oil coming from the deposit of interest for 5 days at the temperature of interest and at a pressure of 140 ± 5 psi, in a high pressure cell.

5) Drain the nuclei impregnated with dead oil at atmospheric-temperature and -pressure until there is no dripping. The draining process lasts around 12 hours and for this purpose a wire mesh of number 200 is used.

6) Weigh the cores impregnated with dead oil and obtain by weight difference the amount of oil that adsorbed the porous medium.

7) Prepare 500 ml of aqueous solution (brine) to evaluate the concentration of chemical required in the test.

8) Place the nuclei impregnated with the dead oil in the Amott cells and carefully add 350 ml of the high salinity brine without chemical and in another cell with the chemical solution to be evaluated at the required concentration.

9) Increase the temperature of the system and maintain it for the period of time to which it is intended to determine the recovery factor under the conditions of temperature and salinity.

10) Quantify the amount of oil produced due to water spontaneous imbibition processes under the study conditions and determine the recovery factor according to the following equation (1):

$$F_r = \left(\frac{A_r}{A_{omp}}\right) \times 100$$

$F_r$ = Recovery factor

$A_r$ = Recovered oil

$A_{omp}$ = Original oil adsorbed in the porous medium

**Example 8**

**Evaluation of a spontaneous imbibition process by wettability change using a light crude oil.** Following the methodology described above, were placed in Amott cells carbonated cores that were saturated with light oil (whose characteristics are shown in table 5) and were put in contact with product solutions 1, 2 and 3 in brine 1 (whose characteristics are shown in table 6). The experiment lasted for 15 days at 90 °C.

**Table 5.** Data of SARA analysis, acid and basic number of light oil.

| Oil | SARA | | | | Total Acid Number (TAN) | Total Basic Number (TBN) |
|---|---|---|---|---|---|---|
| | Satured | Aromatic | Resins | Asphaltenes | | |
| **Light oil** | 30.68 | 28.62 | 39.35 | 1.32 | 0.21 | 1.7 |

**Table 6.** Physico-chemical analysis of the brine 1.

| **Physical properties** | | |
|---|---|---|
| Temperature | 20° C | |
| pH | 7.65 | @ 20°C |
| Density | 1.0043 | g/cm$^3$ @ 20° C |

(continued)

| Physical properties | | | | | |
|---|---|---|---|---|---|
| Conductivity | | μS/cm @ 20° C | | | |
| Turbidity | 4 FTU | | | | |
| **Chemical properties** | | | | | |
| Cations | (mg/L) | (meq/L) | **Anions** | (mg/L) | (meq/L) |
| Sodium ($Na^+$) | 1 703.66 | 74.116 | **Chlorides ($Cl^-$)** | 3 200.00 | 90.260 |
| Potassium ($K^+$) | ----- | ----- | **Sulphates ($SO_4^-$)** | 350.00 | 7.287 |
| Calcium ($Ca^{++}$) | 416.00 | 20.758 | **Carbonates ($CO_3^-$)** | 0.00 | 0.00 |
| Magnesium ($Mg^{++}$) | 106.95 | 8.799 | **Bicarbonates ($HCO_3^-$)** | 405.04 | 6.638 |
| Iron ($Fe^{++}$) | 0.06 | 0.002 | **Hydroxides ($OH^-$)** | ----- | ----- |
| Manganese ($Mn^{++}$) | ----- | ----- | **Nitrites ($NO_2^-$)** | ----- | ----- |
| Barium ($Be^{++}$) | 35.00 | 0.510 | **Nitrates ($NO_3^-$)** | ----- | ----- |
| Strontium ($Sr^{++}$) | ----- | ----- | **Phosphates ($PO_4^{-3}$)** | ----- | ----- |
| **Total:** | **2 261.88** | **104.186** | **Total:** | **3 955.04** | **104.186** |
| **Dissolved and suspended solids** | | | | | |
| | | (mg/L) | | | (mg/L) |
| Total solids | | ----- | Total hardness as $CaCO_3$ | | 1 480,00 |
| Total Dissolved Solids (TDS) | | 6 216,92 | Calcium hardness as $CaCO_3$ | | 1 040,00 |
| Total Dissolved Solids (SST) | | ----- | Magnesium hardness as $CaCO_3$ | | 440,00 |
| Fats and oils | | ----- | Alkalinity to the "F" as $CaCO_3$ | | 0,00 |
| Soluble silica | | ----- | Alkalinity to the "M" as $CaCO_3$ | | 332,00 |
| Ferric oxide | | ----- | Salinity as NaCl | | 5 275,00 |
| Acidity as | | ----- | Stability Index | | 0,288 10 |
| | | | Tendency | | **Encrusting** |

Table 7 shows the recovery data obtained in the Amott cells for products 1, 2 and 3 at concentration of 300 mg/L. Brine 1 was used without additives as reference.

**Table 7.** Light oil recovery results in Amott cells.

| Product | Grams of oil impregnated | Grams of Total Oil Recovered | Percent recovery |
|---|---|---|---|
| Reference | 12.2874 | 4.5796 | 37.27 |
| 1 | 12.1547 | 6.3253 | 52.04 |
| 2 | 11.8760 | 6.0987 | 51.35 |
| 3 | 11.5244 | 5.6878 | 49.35 |

From the results of Table 7 it is possible to observe that the branched geminal zwitterionic liquids, hidroxisultaine- and sulfobetaine-base (Products 1, 2 and 3) recover about 1.5 times more, light crude oil than the reference therefore functioning properly as wettability modifiers in spontaneous imbibition processes.

**Example 9**

**Evaluation of a spontaneous imbibition process due to wettability change using a heavy crude oil.** According to the methodology described above, were placed in the Amott cells carbonated cores saturated with heavy

oil, in contact with the product solutions 1 and 2 in the brine 2 at a concentration of 300 mg/L.

The characteristics of heavy crude oil and brine 2 are shown in Tables 8 and 9, respectively.

**Table 8.** SARA analysis data, acid and basic total number of heavy crude oil.

| Oil | SARA | | | | Total acid number (TAN) | Total basic number (TBN) |
|---|---|---|---|---|---|---|
| | Saturated | Aromatic | Resins | Asphaltenes | | |
| Heavy oil | 13.4 | 24.76 | 51.01 | 10.44 | 1.83 | 2.12 |

**Tabla 9.** Physico-chemical analysis of the brine 2.

| Physical properties | | | | | |
|---|---|---|---|---|---|
| Temperature | 20 °C | | | | |
| pH | 6.68 | @ 20°C | | | |
| Density | 1.0216 | g/cm$^3$ @ 20 °C | | | |
| Conductivity | 45,600 | μS/em @ 20 °C | | | |
| Turbidity | 15 FTU | | | | |
| **Chemical properties** | | | | | |
| Cations | (mg/L) | (meq/L) | **Anions** | (mg/L) | (meq/L) |
| Sodium ($Na^+$) | 11,630.06 | 505.907 | **Chlorides ($Cl^-$)** | 22,000.00 | 620.540 |
| Potassium ($K^+$) | ----- | ----- | **Sulphates ($SO_4^=$)** | 825.00 | 17.177 |
| Calcium ($Ca^{++}$) | 1,976.00 | 98.603 | **Carbonates ($CO_3^=$)** | 0.00 | 0.00 |
| Magnesium ($Mg^{++}$) | 427.86 | 35.197 | **Bicarbonates ($HCO_3^-$)** | 122.00 | 1.999 |
| Iron ($Fe^{++}$) | 0.25 | 0.009 | **Hydroxides ($OH^-$)** | ----- | ----- |
| Manganese ($Mn^{++}$) | ----- | ----- | **Nitrites ($NO_2^-$)** | ----- | ----- |
| Barium ($Ba^{++=)}$) | ----- | ----- | **Nitrates ($NO_3^-$)** | ----- | ----- |
| Strontium ($Sr^{++}$) | ----- | ----- | **Phosphates ($PO_4^{-3}$)** | ----- | ----- |
| Total: | **14,034.41** | **639.716** | **Total:** | **22,947.00** | **639.716** |
| **Dissolved and suspended solids** | | | | | |
| | | (mg/L) | | | (mg/L) |
| Total solids | | ----- | Total hardness as $CaCO_3$ | | 6,700.00 |
| Total Dissolved Solids (TDS) | | 36,981.41 | Calcium hardness as $CaCO_3$ | | 4,940.00 |
| Total Dissolved Solids (TSS) | | ----- | **Magnesium hardness as $CaCO_3$** | | 1,760.00 |
| Fats and oils | | ----- | **Alkalinity to the "F" as $CaCO_3$** | | 0.00 |
| Soluble silica | | ----- | **Alkalinity to the "M" as $CaCO_3$** | | 100.00 |
| Ferric oxide | | ----- | Salinity as NaCl | | 36,265.59 |
| Acidity as | | ----- | Stability Index | | -0.71714 |
| | | | Tendency | | **Corrosive** |

Table 10 shows the obtained results in the Amott cells with carbonate core in contact with dissolutions of geminal zwitterionic liquids, sulfobetaine and hidroxisultaine-type (products 1 and 2) at a concentration of 300 mg/L in the brine 2 and using heavy crude oil.

**Table 10.** Heavy oil recovery results in Amott cells.

| Product | Grams of oil impregnated | Total grams of oil recovered | Percentage of recovery (%) |
|---|---|---|---|
| Reference | 6.8124 | 0.8364 | 12.2776 |
| 1 | 6.2834 | 2.4394 | 38.8229 |
| 2 | 6.3646 | 1.1320 | 17.8419 |

The results of table 10, it is possible to observe that the zwitterionic liquid, sulfobetaine-base (Product 1) retrieves more than three times more crude oil with respect to the reference, and the zwitterionic liquid, hidroxisultaine-base recovers about 1.5 times more oil that reference the branched geminales zwitteriónicos liquid, sulfobetaine and hidroxisultaine-base (Product 2), so it work properly as wettability modifiers on spontaneous imbibition processes.

**Determination of the efficiency of corrosion inhibition through the method NACE 1D-182.** It is a gravimetric test commonly called dynamic wheel (Wheel test) that simulates the corrosive medium characteristic of oil production, is a dynamic procedure developed for fluids (oil, water and inhibitor).

**Equipment and reagents for tests.**

a) Dynamic evaluator for corrosion inhibitors with temperature controller, 30-rpm stirrer speed and capacity for 52 bottles of 180 ml.
b) 200-ml bottles.
c) SAE 1010 carbon steel coupons having 1"× 0.5" × 0.010" dimensions.
d) Glassware for the preparation of the corrosive environment. It consists of a 2L glass reactor equipped with a cooling bath, a mechanical stirrer, a bubbler for gas (nitrogen and hydrogen sulfide), and has an outlet joined to two in series-connected traps (the first one is charged with sodium hydroxide in pellet form and the second one with a 20% sodium hydroxide solution), so hydrogen sulfide does not contaminate the environment.
e) Potentiometer for pH measuring.

The test conditions are shown in Table 11.

**Table 11.** Test conditions.

| | |
|---|---|
| Temperature | 60 °C |
| Aqueous medium | Synthetic brine with 600 ± 50 ppm of $H_2S$ |
| Test time | 46 hours |
| Organic medium | Kerosene |
| Brine/organic medium volume ratio | 90/10 |
| Test volume | 180 mL |
| Medium pH | 4 |
| Corrosion witness (metal coupons) | SAE 1010 steel |

The composition of brine 3 is shown in Table 12.

**Table 12.** Composition of the brine 3.

| Salts | Quantity (g/L) |
|---|---|
| NaCl | 60.0 |
| $CaCl_2 \cdot H_2O$ | 6.0 |
| $MgCl_2 \cdot 6H_2O$ | 10.48 |
| $Na_2SO_4$ | 3.5 |

**Getting results.** The weight difference of the coupons before and after they have been exposed to the corrosive liquid during 46 hours is a direct indication of the metal lost due to the corrosion.

The efficiency as a corrosion inhibitor is established by comparing the corrosion velocities of the witness or blank with the velocity of the witness having a determined corrosion inhibitor concentration, through the following formula (2):

$$Efficiency\ (\%) = \frac{(V_0 - V)}{V_0} \times 100 \qquad (2)$$

where:

$V_0$ = The corrosion velocity of the reference coupon (blank).
$V$ = The corrosion velocity of the coupon having the corrosion inhibitor.

Table 13 shows the results for the Products 1 and 2 used at different concentrations.

**Table 13.** Results of the corrosion rate and efficiency.

| Product | Concentration (ppm) | Corrosion velocity *(mpy) | Efficiency (%) |
|---|---|---|---|
| Reference | 0 | 32.9 | 0 |
| 1 | 50 | 2.8 | 91.4 |
| 1 | 100 | 2.0 | 93.8 |
| 2 | 50 | 2.9 | 90.9 |
| 2 | 100 | 2.8 | 91.4 |
| *mpy: thousandths of an inch per year | | | |

From results of Table 13, it is possible to observe that the zwitterionic liquid, sulfobetaine-base (Product 1) and the zwitterionic liquid, hidroxisultaine-base (Product 2) behave properly as corrosion inhibitors.

## Claims

1. Branched geminal zwitterionic liquids, sulfobetaine- and hydroxysultaine-base with the following structural formula:

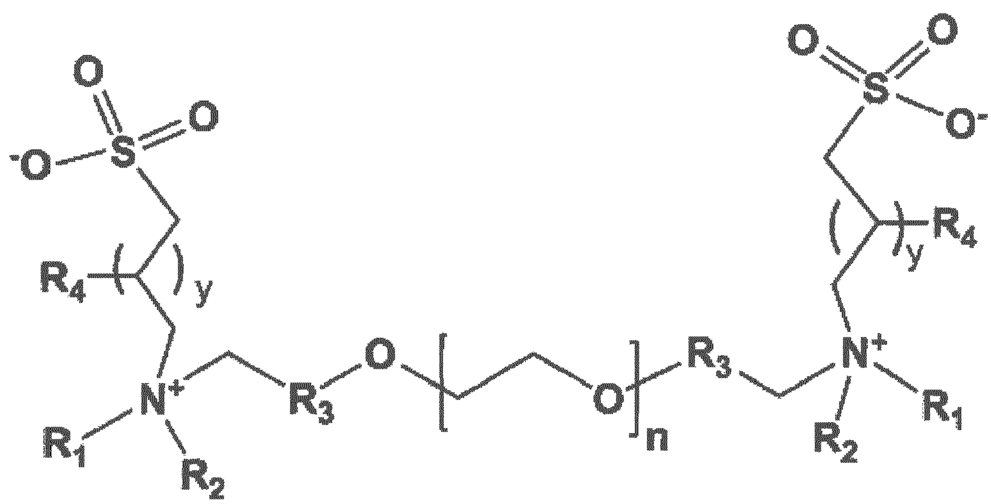

Where:

**$R_1$** = It is a radical represented by alkyl or alkenyl chains, linear or branched; or a group cycle alkyl or aryl;

**$R_2$** = It is a radical represented by alkyl or alkenyl chains, linear or branched; or a group cycle alkyl or aryl;
**$R_3$** = $-CH_2-$, $-CH_2-CH(OH)-$;
y = values of 0 and 1;

When is y equal to 1:

**$R_4$** = It is a radical represented by -OH;
**n** values of 1 to 500.

2. A process to prepare compounds according to claim 1, **characterized in that** it is represented by the following synthetic schemes:

Route 1

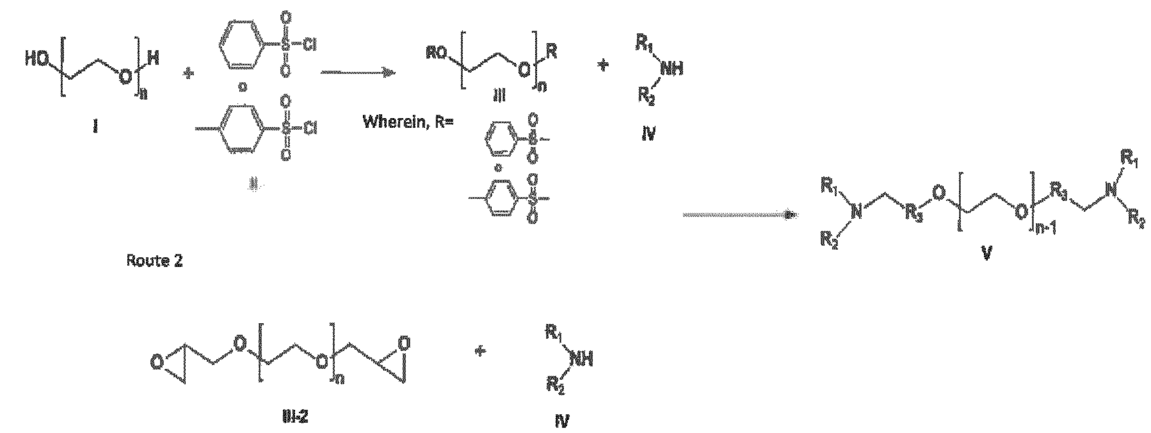


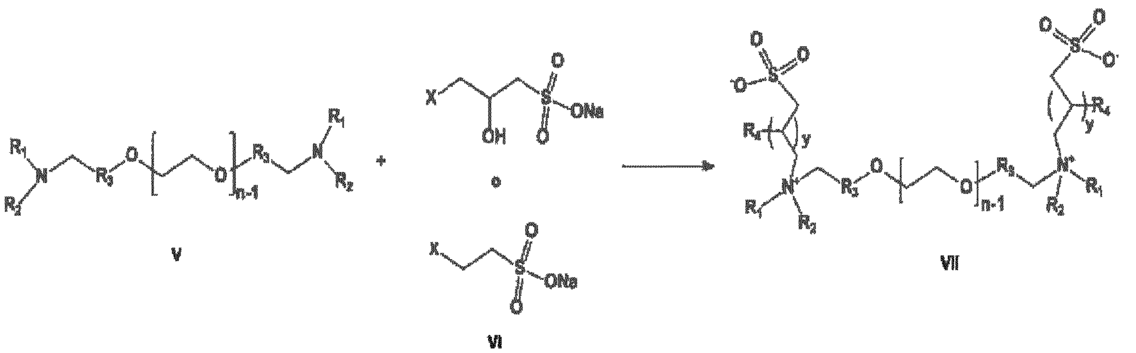


Wherein, X= Cl, Br and comprising two synthesis routes: Route 1: The first step consists in to react polyethylene glycols of the formula **I** with benzenesulfonyl chloride or tosyl chloride of the formula **II**; The second step consists in to react the compounds of the formula **III** through a nucleophilic substitution with primary or secondary amines of the formula **IV;** so it is obtained the tertiary amines of formula **V;** The third step consists in to obtain the geminal zwitterionic liquids, sulfobetaine and hydroxysultaine -based of the formula **VII;,** which are obtained by reacting the tertiary amines of the formula V with sodium 2-bromoethane sulfonate or sodium or 3-chloro-2-hydroxypropane sodium sulfonate of the formula **VI.;** and **route 2:** The first step consists in to react polyethylene glycol diglycidyl ether of the formula **I** with primary or secondary amines of the formula **IV.** The second step consists in to react the aminoalcohols of the formula **V** with 2-bromoethane ethane sodium sulfonate or sodium 3-chloro-2-hydroxy-propane sodium sulfonate of the formula **VI** to obtain the geminal zwitterionic liquids, sulfobetaine and hydroxysultaine-based of the formula **VII.**

3. The process according to claim 2, wherein the molecular weight of the polyethylene glycols of formula I used in the synthesis route 1 is in the range from 100 to 20,000 g/mol.

4. The process according to claim 2, wherein the shown reaction in route 1 between the polyethylene glycols of formula I with benzenesulfonyl chloride or tosyl chloride of formula II takes place with a relationship molar from 1:1 to 1:**4**.

**5.** The process according to claim 4, wherein the reaction is carried out in a basic medium composed of sodium, potassium or cesium hydroxide.

**6.** The process according to claim 4, wherein in the reaction water, tetrahydrofuran, acetonitrile, chloroform or mixtures of these are used as a solvent.

**7.** The process according to claims 2 and 4, wherein to form the compounds of formula III of route **1** of synthesis, the reaction is carried out in a reaction time of 1 to 12 hours.

**8.** The process according to claims 2 and 4, wherein to form the compounds of formula III of route **1** of synthesis, the reaction is carried out at a temperature of 0 ° c to 25 ° C.

**9.** The process according to claim 2, wherein the reaction shown on the synthesis route 1, is carried out with a molar ratio between formula III compounds and secondary amines of formula IV from 1:1.5 to 1:4.

**10.** The process according to claims 2 and 9, wherein the secondary amines of formula IV whose $R_1$ and $R_2$ are linear or branched alkyl or alkenyl chains, preferably of 1 to 30 carbon atoms; or cyclic alkyl or aryl groups.

**11.** The process according to claims 2 and 9, wherein the reaction shown on the synthesis route 1 for the preparation of tertiary amines of formula V, is carried out in the presence of solvents, such as acetonitrile, chloroform, dimethylformamide, dimethyl sulfoxide, acetone or short-chain alcohols.

**12.** The process according to claims 2 and 9, wherein the reaction shown on the synthesis route 1 for the preparation of tertiary amines of formula V, is carried out in a reaction time of 5 to 60 hours.

**13.** The process according to claims 2 and 9, wherein the reaction shown on the synthesis route 1 for the preparation of tertiary amines of formula V, is carried out at a temperature of 50 °C to 150 °C.

**14.** The process according to claims 2, wherein the reaction shown on the synthesis route 1 among the compounds of formula V with 2-bromo ethane sodium sulfonate or 3-chlorine-2-hydroxy-propane sodium sulfonate of formula VI, is carried out with molar ratio from 1:1.5 to 1:4.

**15.** The process according to claims 2 and 14, wherein the reaction shown on the synthesis route 1 for the preparation of the compounds of formula VII is carried out in the presence of solvents such as water, alcohols, aromatics and inert hydrocarbons.

**16.** The process according to claims 2 and 14, wherein the reaction time, the temperature and the pressure for the preparation of the compounds of formula VII shown on the synthesis route 1, depend on the structure of the compounds of formula V and VI.

**17.** The process according to claim 16, wherein reaction time varies from 6 to 72 hours.

**18.** The process according to claim 16, wherein the temperature is from 40 to 180 °C.

**19.** The process according to claim 16, wherein the pressure varies from 779 to 1013 hPa (585 to 760 mmHg) and is preferably atmospheric.

**20.** The process according to claim 2, wherein the polyethylene glycols of formula I used in the route of synthesis 2 contain two epoxide groups, one at the end and the other at the beginning of the polymer chain.

**21.** The process according to claim 2, wherein the molecular weight of the polyethylene glycols of formula I used in the route of synthesis 2 is selected in the range from 100 to 22,000 g/mol.

**22.** The process according to claim 2, wherein the reaction described in the synthesis route 2 between the compounds of formula I and primary or secondary amines of formula II is at a molar ratio from 1:1.5 to 1:4.

**23.** The process according to claims 2 and 4, wherein in the primary and secondary amines used in the route of synthesis 2 of formula IV, whose $R_1$ and $R_2$ are linear or branched alkyl or alkenyl chains, having preferably from 1 to 30

carbon atoms; or cyclic alkyl or aryl groups.

**24.** The process according to claims 2 and 4, wherein the reaction shown on the route of synthesis 2 for the preparation of the amino alcohols of formula V, is carried out in the presence of solvents such as acetonitrile, dioxane, chloroform, dimethylformamide, dimethyl sulfoxide, acetone or short-chain alcohols.

**25.** The process according to claims 2 and 4, wherein the reaction described in the route of synthesis 2 for the preparation of the amino alcohols of formula V, is conducted in a reaction time from 6 to 48 hours, preferably from 12 to 20 hours.

**26.** The process according to claims 2 and 4, wherein the reaction described in the route of synthesis 2 for the preparation of the amino alcohols of formula V, is carried out at a temperature of 50 °C to 150 °C, preferably from 60 to 90 °C.

**27.** The process according to claim 2, wherein the reaction described in the synthesis route 2 between the compounds of formula V with 2-bromo ethane sodium sulfonate or 3-chlorine-2-hydroxy-propane sodium sulfonate of formula VI, is carried out with a molar ratio of 1:1.5 to 1:4.

**28.** The process according to claims 2 and 27, wherein the reaction described in the synthesis route 2 to obtain the compounds of formula VII is carried out in in the presence of solvents such as water, dioxane, alcohols, aromatics or hydrocarbons.

**29.** The process according to claim 27, wherein the reaction time varies from 6 to 48 hours.

**30.** The process according to claim 27, wherein the temperature is from 40 to 180 °C.

**31.** The process according to the claim 27, wherein the pressure varies from 779 to 1013 hPa (585 to 760 mmHg) and is preferably atmospheric.

**32.** Use of geminal zwitterionic liquid, sulfobetaine- and hydroxysultaine-base, of structural formula described in claim **1** in enhanced oil recovery processes, wherein the wettability of rocks such as limestone, dolomite, sandstone, quartz or heterogeneous lithologies is modified.

**33.** The use of geminal zwitterionic liquids according to claim 32, in enhanced oil recovery processes, wherein there is the presence of brine with high content of salts and divalent ions such as calcium, magnesium, barium and strontium.

**34.** The use of geminal zwitterionic liquids according to claim 32, wherein the temperature is up to 220°C.

**35.** The use of geminal zwitterionic liquids according to claim 32, wherein the pressure is up to 5.516•107 Pa (8000 psi).

**36.** The use of geminal zwitterionic liquids according to claim 32, wherein the concentration of salts is up to 400,000 ppm.

**37.** The use of geminal zwitterionic liquids according to claim 32, wherein the concentration of divalent ions is up to 180,000 ppm.

**38.** The use of geminal zwitterionic liquids according to claim 32, wherein the concentration to inject of the zwitterionic liquid in order to modify the wettability of oil-rock is in concentrations of 25 to 40,000 ppm.

**39.** The use of geminal zwitterionic liquids according to claim 38, wherein the concentration to inject is from 500 to 10,000 ppm.

**40.** The use of geminal zwitterionic liquids of structural formula according to claim 1, as corrosion inhibitors wherein the corrosion of ferrous metals used in pipelines and equipment for operations of extraction and transport of crude oil is inhibited.

**41.** The use of geminal zwitterionic liquids according to claim 40, wherein the concentration to inject of the zwitterionic liquid in order to inhibit corrosion is from 25 to 500 ppm.

**42.** The use of geminal zwitterionic liquids according to claim 41, wherein the concentration to inject it is selected from 50 to 300 ppm.

## Patentansprüche

1. Verzweigte geminale zwitterionische Flüssigkeiten auf Basis von Sulfobetain- und Hydroxysultain mit der folgenden Strukturformel:

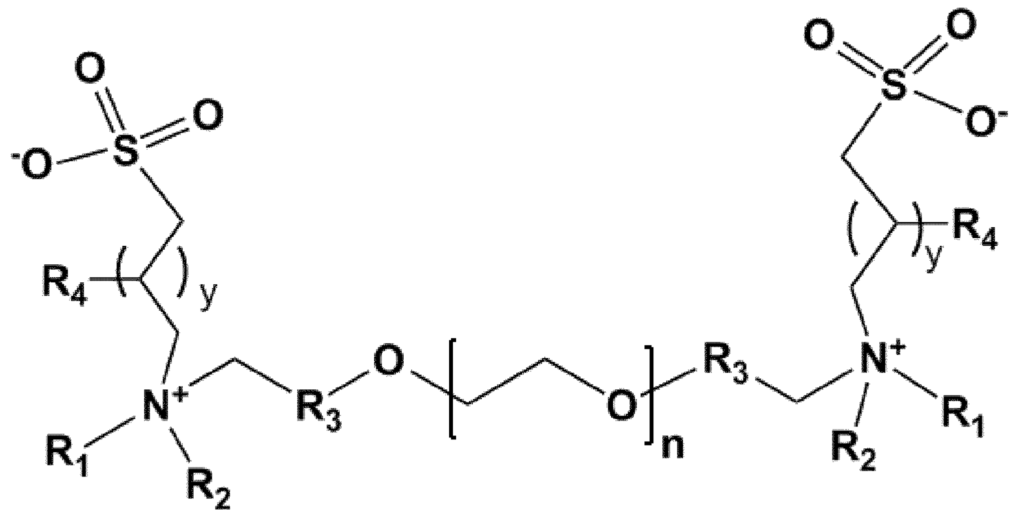

wobei:

es sich bei $R_1$ um einen Rest handelt, der durch Alkyl- oder Alkenylketten, linear oder verzweigt, oder durch eine cyclische Alkyl- oder Arylgruppe dargestellt wird;
es sich bei $R_2$ um einen Rest handelt, der durch Alkyl- oder Alkenylketten, linear oder verzweigt, oder durch eine cyclische Alkyl- oder Arylgruppe dargestellt wird;
$R_3$ = $-CH_2-$, $-CH_2-CH(OH)-$ ist;
y Werte von o und 1 annimmt;

wenn y gleich 1 ist:

$R_4$ = ein Radikal ist, das durch -OH dargestellt wird; und
n Werte von 1 bis 500 annimmt.

2. Verfahren zur Herstellung von Verbindungen nach Anspruch 1, **dadurch gekennzeichnet, dass** es durch die folgenden Syntheseschemata dargestellt wird:

Route 1

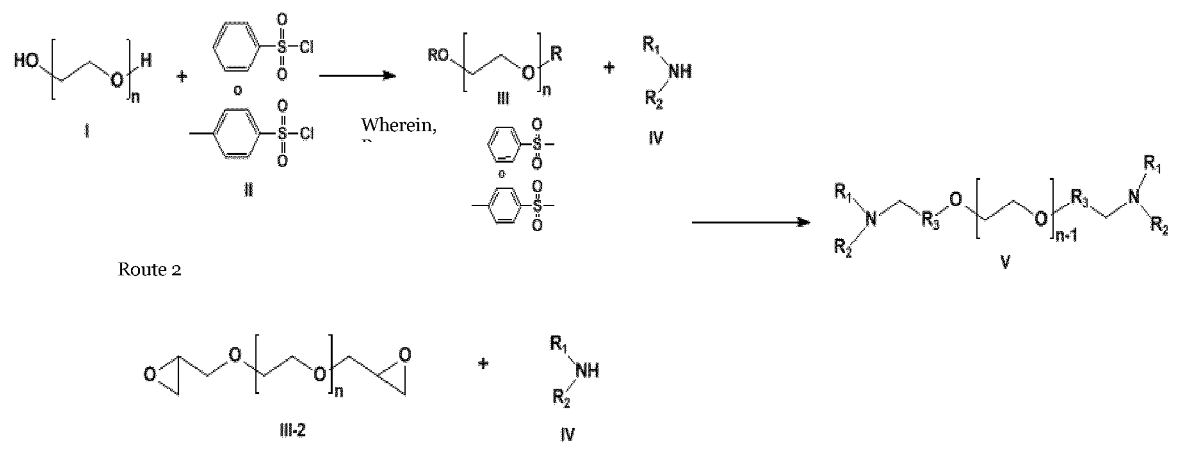


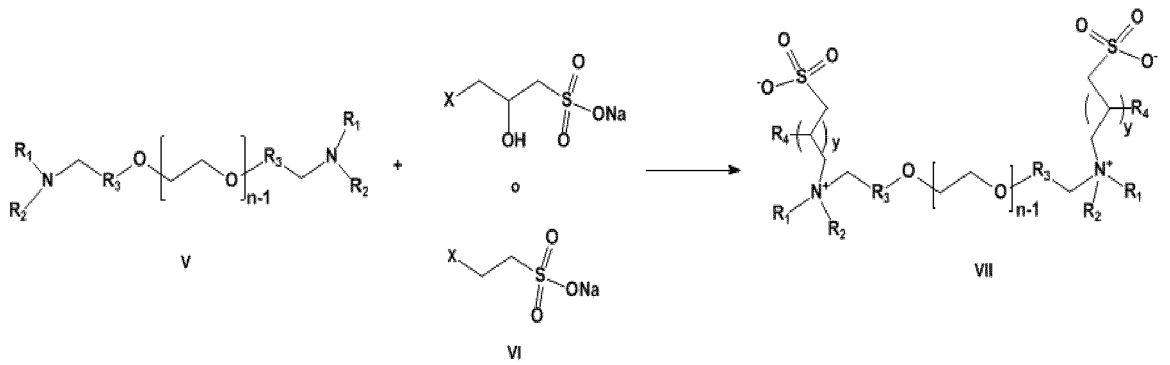

Wherein, X= Cl, Br und zwei Synthesewege umfasst:

Route 1: Der erste Schritt besteht darin, Polyethylenglycole der Formel I mit Benzolsulfonylchlorid oder Tosylchlorid der Formel II umzusetzen; der zweite Schritt besteht darin, die Verbindungen der Formel III durch eine nukleophile Substitution mit primären oder sekundären Aminen der Formel IV umzusetzen; so dass die tertiären Amine der Formel V erhalten werden; der dritte Schritt besteht darin, die geminalen zwitterionischen Flüssigkeiten auf Basis von Sulfobetain- und Hydroxysultain der Formel VII zu erhalten, die durch Umsetzung der tertiären Amine der Formel V mit Natrium-2-bromethansulfonat oder Natrium oder Natrium-3-chlor-2-hydroxypropansulfonat der Formel VI erhalten werden; und

Route 2: Der erste Schritt besteht darin, Polyethylenglycoldiglycidylether der Formel I mit primären oder sekundären Aminen der Formel IV umzusetzen; der zweite Schritt besteht darin, die Aminoalkohole der Formel V mit Natrium-2-bromethansulfonat oder Natrium-3-chlor-2-hydroxypropansulfonat der Formel VI umzusetzen, um die geminalen zwitterionischen Flüssigkeiten auf Basis von Sulfobetain- und Hydroxysultain der Formel VII zu erhalten.

3. Verfahren nach Anspruch 2, bei dem das Molekulargewicht der im Syntheseroute 1 verwendeten Polyethylenglycole der Formel I im Bereich von 100 bis 20.000 g/mol liegt.

4. Verfahren nach Anspruch 2, bei dem die gezeigte Reaktion in Route 1 zwischen den Polyethylenglycolen der Formel I mit Benzolsulfonylchlorid oder Tosylchlorid der Formel II in einem molaren Verhältnis von 1:1 bis 1:4 erfolgt.

5. Verfahren nach Anspruch 4, bei dem die Reaktion in einem basischen Medium zusammengesetzt aus Natrium-, Kalium- oder Cäsiumhydroxid durchgeführt wird.

6. Verfahren nach Anspruch 4, bei dem für die Reaktion Wasser, Tetrahydrofuran, Acetonitril, Chloroform oder Mischungen davon als Lösungsmittel verwendet werden.

7. Verfahren nach einem der Ansprüche 2 und 4, bei dem zur Bildung der Verbindungen der Formel III der Syntheseroute

1 die Reaktion in einer Reaktionszeit von 1 bis 12 Stunden durchgeführt wird.

8. Verfahren nach einem der Ansprüche 2 und 4, bei dem zur Bildung der Verbindungen der Formel III der Syntheseroute 1 die Reaktion bei einer Temperatur von 0°C bis 25°C durchgeführt wird.

9. Verfahren nach Anspruch 2, bei dem die auf der Syntheseroute 1 gezeigte Reaktion mit einem molaren Verhältnis zwischen Verbindungen der Formel III und sekundären Aminen der Formel IV von 1:1,5 bis 1:4 durchgeführt wird.

10. Verfahren nach einem der Ansprüche 2 und 9, bei dem $R_1$ und $R_2$ der sekundären Amine der Formel IV lineare oder verzweigte Alkyl- oder Alkenylketten, vorzugsweise mit 1 bis 30 Kohlenstoffatomen; oder cyclische Alkyl- oder Arylgruppen sind.

11. Verfahren nach einem der Ansprüche 2 und 9, bei dem die auf der Syntheseroute 1 gezeigte Reaktion zur Herstellung von tertiären Aminen der Formel V in Gegenwart von Lösungsmitteln, wie Acetonitril, Chloroform, Dimethylformamid, Dimethylsulfoxid, Aceton oder kurzkettigen Alkoholen durchgeführt wird.

12. Verfahren nach einem der Ansprüche 2 und 9, bei dem die auf der Syntheseroute 1 gezeigte Reaktion zur Herstellung von tertiären Aminen der Formel V in einer Reaktionszeit von 5 bis 60 Stunden durchgeführt wird.

13. Verfahren nach einem der Ansprüche 2 und 9, bei dem die auf der Syntheseroute 1 gezeigte Reaktion zur Herstellung von tertiären Aminen der Formel V bei einer Temperatur von 50 °C bis 150 °C durchgeführt wird.

14. Verfahren nach Anspruch 2, bei dem die auf der Syntheseroute 1 gezeigte Reaktion der Verbindungen der Formel V mit Natrium-2-bromethansulfonat oder Natrium-3-chlor-2-hydroxy-propansulfonat der Formel VI in einem Molverhältnis von 1:1,5 bis 1:4 durchgeführt wird.

15. Verfahren nach einem der Ansprüche 2 und 14, bei dem die auf der Syntheseroute 1 gezeigte Reaktion zur Herstellung der Verbindungen der Formel VII in Gegenwart von Lösungsmitteln wie Wasser, Alkoholen, Aromaten und inerten Kohlenwasserstoffen durchgeführt wird.

16. Verfahren nach einem der Ansprüche 2 und 14, bei dem die Reaktionszeit, die Temperatur und der Druck für die Herstellung der Verbindungen der Formel VII, die auf der Syntheseroute 1 dargestellt sind, von der Struktur der Verbindungen der Formel V und VI abhängen.

17. Verfahren nach Anspruch 16, bei dem die Reaktionszeit von 6 bis 72 Stunden variiert.

18. Verfahren nach Anspruch 16, bei dem die Temperatur von 40 °C bis 180 °C beträgt.

19. Verfahren nach Anspruch 16, bei dem der Druck von 779 hPa bis 1013 hPa (585 bis 760 mmHg) variiert und vorzugsweise atmosphärisch ist.

20. Verfahren nach Anspruch 2, bei dem die Polyethylenglycole der Formel I, die auf der Syntheseroute 2 verwendet werden, zwei Epoxidgruppen enthalten, eine am Ende und die andere am Anfang der Polymerkette.

21. Verfahren nach Anspruch 2, bei dem das Molekulargewicht der auf der Syntheseroute 2 verwendeten Polyethylenglycole der Formel I aus dem Bereich von 100 bis 22.000 g/mol ausgewählt ist.

22. Verfahren nach Anspruch 2, bei dem die in Syntheseroute 2 beschriebene Umsetzung zwischen den Verbindungen der Formel I und primären oder sekundären Aminen der Formel II in einem Molverhältnis von 1:1,5 bis 1:4 erfolgt.

23. Verfahren nach einem der Ansprüche 2 und 4, bei dem in den in der Syntheseroute 2 verwendeten primären und sekundären Aminen der Formel IV deren $R_1$ und $R_2$ lineare oder verzweigte Alkyl- oder Alkenylketten, vorzugsweise mit 1 bis 30 Kohlenstoffatomen, oder cyclische Alkyl- oder Arylgruppen sind.

24. Verfahren nach einem der Ansprüche 2 und 4, bei dem die auf der Syntheseroute 2 gezeigte Reaktion zur Herstellung der Aminoalkohole der Formel V in Gegenwart von Lösungsmitteln wie Acetonitril, Dioxan, Chloroform, Dimethylformamid, Dimethylsulfoxid, Aceton oder kurzkettigen Alkoholen durchgeführt wird.

25. Verfahren nach einem der Ansprüche 2 und 4, bei dem die in Syntheseroute 2 beschriebene Reaktion zur Herstellung der Aminoalkohole der Formel V in einer Reaktionszeit von 6 bis 48 Stunden, vorzugsweise von 12 bis 20 Stunden, durchgeführt wird.

26. Verfahren nach einem der Ansprüche 2 und 4, bei dem die in Syntheseroute 2 beschriebene Reaktion zur Herstellung der Aminoalkohole der Formel V bei einer Temperatur von 50 °C bis 150 °C, vorzugsweise von 60 bis 90 °C, durchgeführt wird.

27. Verfahren nach Anspruch 2, bei dem die in Syntheseroute 2 beschriebene Umsetzung der Verbindungen der Formel V mit Natrium-2-bromethansulfonat oder Natrium-3-chlor-2-hydroxypropansulfonat der Formel VI in einem Molverhältnis von 1:1,5 bis 1:4 durchgeführt wird.

28. Verfahren nach einem der Ansprüche 2 und 27, bei dem die in Syntheseroute 2 beschriebene Reaktion zur Gewinnung der Verbindungen der Formel VII in Gegenwart von Lösungsmitteln wie Wasser, Dioxan, Alkoholen, Aromaten oder Kohlenwasserstoffen durchgeführt wird.

29. Verfahren nach Anspruch 27, bei dem die Reaktionszeit im Bereich von 6 bis 48 Stunden variiert.

30. Verfahren nach Anspruch 27, bei dem die Temperatur 40 °C bis 180 °C beträgt.

31. Verfahren nach Anspruch 27, bei dem der Druck von 779 hPa bis 1013 hPa (585 bis 760 mmHg) variiert und vorzugsweise atmosphärisch ist.

32. Verwendung einer geminalen zwitterionischen Flüssigkeit auf Basis von Sulfobetain- und Hydroxysultain mit der in Anspruch 1 beschriebenen Strukturformel in Verfahren zur verbesserten Ölgewinnung, bei der die Benetzbarkeit von Gesteinen wie Kalkstein, Dolomit, Sandstein, Quarz oder heterogenen Lithologien modifiziert wird.

33. Verwendung von geminalen zwitterionischen Flüssigkeiten nach Anspruch 32 in verbesserten Ölgewinnungsprozessen, bei der eine Sole mit hohem Gehalt an Salzen und zweiwertigen Ionen wie Calcium, Magnesium, Barium und Strontium vorliegt.

34. Verwendung von geminalen zwitterionischen Flüssigkeiten nach Anspruch 32, bei der die Temperatur bis zu 220°C beträgt.

35. Verwendung von geminalen zwitterionischen Flüssigkeiten nach Anspruch 32, bei der der Druck bis zu $5{,}516*10^7$ Pa (8000 psi) beträgt.

36. Verwendung von geminalen zwitterionischen Flüssigkeiten nach Anspruch 32, bei der die Konzentration der Salze bis zu 400.000 ppm beträgt.

37. Verwendung von geminalen zwitterionischen Flüssigkeiten nach Anspruch 32, bei der die Konzentration an zweiwertigen Ionen bis zu 180.000 ppm beträgt.

38. Verwendung von geminalen zwitterionischen Flüssigkeiten nach Anspruch 32, bei der die einzuspritzende Konzentration der zwitterionischen Flüssigkeit zur Modifizierung der Benetzbarkeit von Öl-Gestein in Konzentrationen von 25 bis 40.000 ppm liegt.

39. Verwendung von geminalen zwitterionischen Flüssigkeiten nach Anspruch 38, bei der die zu injizierende Konzentration 500 bis 10.000 ppm beträgt.

40. Verwendung von geminalen zwitterionischen Flüssigkeiten der Strukturformel nach Anspruch 1 als Korrosionsinhibitoren, bei der die Korrosion von eisenhaltigen Metallen, die in Pipelines und Ausrüstung für den Betrieb der Gewinnung und des Transports von Rohöl verwendet werden, gehemmt wird.

41. Verwendung von geminalen zwitterionischen Flüssigkeiten nach Anspruch 40, bei der die zu injizierende Konzentration der zwitterionischen Flüssigkeit zur Korrosionshemmung 25 bis 500 ppm beträgt.

42. Verwendung von geminalen zwitterionischen Flüssigkeiten nach Anspruch 41, bei der die einzuspritzende Konzentration aus 50 bis 300 ppm ausgewählt ist.

## Revendications

1. Liquides zwittérioniques géminaux ramifiés, à base de sulfobétaïne et d'hydroxysultaïne de la formule structurale suivante :

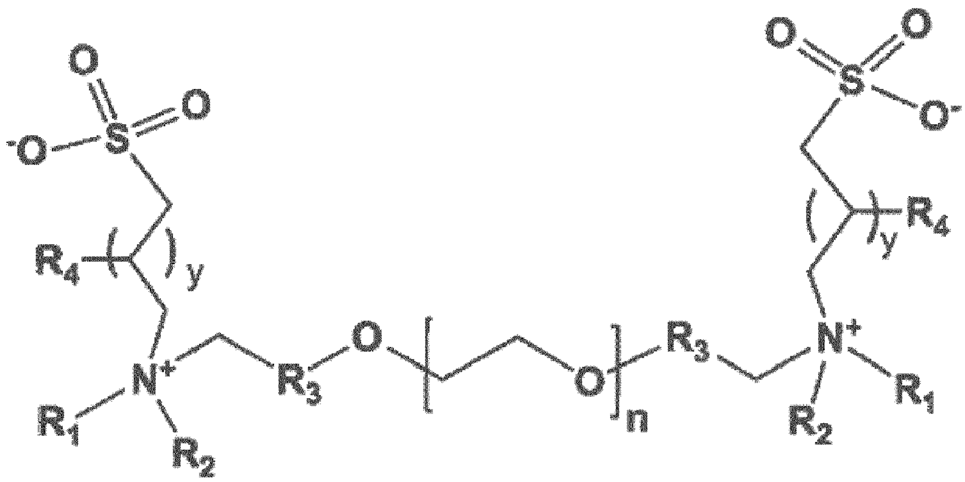

Dans laquelle :

$R_1$ = un radical représenté par des chaînes alkyle ou alcényle, linéaires ou ramifiées ; ou un groupe du cycle alkyle ou aryle ;
**$R_2$** = un radical représenté par des chaînes alkyle ou alcényle, linéaires ou ramifiées ; ou un groupe du cycle alkyle ou aryle ;
$R_3$ = $-CH_2-$, $-CH_2-CH(OH)-$ ;
y = valeurs valant de 0 et 1 ;

Lorsque y est égal à 1 :

**$R_4$** = un radical représenté par -OH ;
n représente des valeurs valant de 1 à 500.

2. Procédé de préparation de composés selon la revendication 1, **caractérisé en ce qu'**il est représenté par les schémas de synthèse suivants :

Voie 1

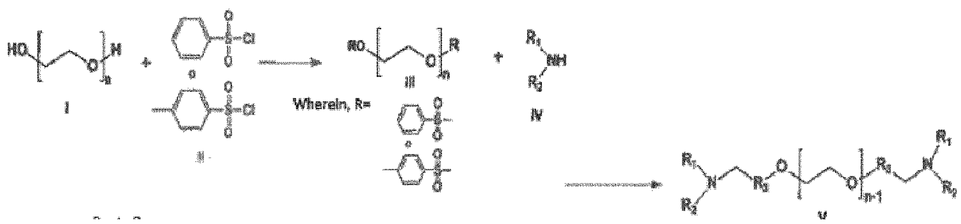


Wherein $R^1$ : Où $R^1$
Voie 2
$R^2$

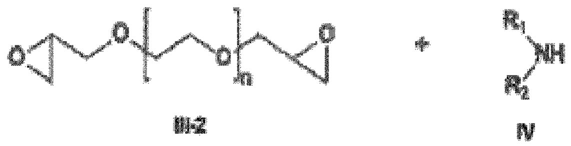

III-2 IV

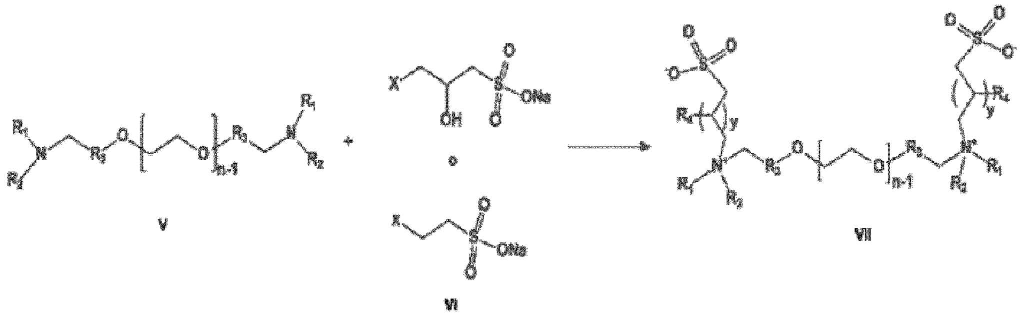

V VI o VII

Wherein X = Cl, Br : Où X = Cl, Br

et comprenant deux voies de synthèse : Voie 1 : La première étape consiste à faire réagir des polyéthylènes glycols de la formule I avec du chlorure de benzènesulfonyle ou du chlorure de tosyle de la formule **II** ; la deuxième étape consiste à faire réagir les composés de la formule **III** par le biais d'une substitution nucléophile avec des amines primaires ou secondaires de la formule **IV** ; on obtient ainsi les amines tertiaires de la formule **V**; la troisième étape consiste à obtenir les liquides zwittérioniques géminaux à base de sulfobétaïne et d'hydroxysultaïne de la formule **VII**; qui sont obtenus en faisant réagir les amines tertiaires de la formule **V** avec du sodium-2-bromoéthanesulfonate ou du sodium ou du sodium-3-chloro-2-hydroxypropanesulfonate de la formule **VI** ; et **Voie 2** ; la première consiste à faire réagir de l'éther diglycidylique de polyéthylène glycol de la formule **I** avec des amines primaires ou secondaires de la formule **IV.** La deuxième étapes consiste è faire réagir les aminoalcools de la formule **V** avec du sodium-2-bromoéthanesulfonate ou du sodium-3-chloro-2-hydroxypropane sulfonate de la formule **VI** pour obtenir des liquides zwittérioniques géminaux à base de sulfobétaïne et d'hydroxysultaïne de la formule **VII.**

**3.** Procédé selon la revendication 2, dans lequel le poids moléculaire des polyéthylènes glycols de la formule I se situe dans la plage allant de 100 à 20,000 g/mol.

**4.** Procédé selon la revendication 2, dans lequel la réaction mise en évidence dans la voie 1 entre les polyéthylène glycols de la formule 1 avec le chlorure de benzènesulfonyle ou le chlorure de tosyle de la formule II a lieu avec un rapport molaire de 1 : 1 et 1 : 4.

**5.** Procédé selon la revendication 4, dans lequel la réaction s'effectue dans un milieu basique composé d'hydroxyde de sodium, de potassium ou de césium.

**6.** Procédé selon la revendication 4, dans lequel l'eau de réaction, le tétrahydrofurane, l'acétonitrile, le chloroforme ou les mélanges de ceux-ci sont utilisés comme solvant.

**7.** Procédé selon les revendications 2 et 4, dans lequel pour former les composés de la formule III de la voie de synthèse 1, la réaction st effectuée en un temps de réaction de 1 à 12 heures.

**8.** Procédé selon les revendications 2 et 4, dans lequel pour former les composés de la formule III de la voie de de synthèse 1, la réaction est effectuée à une température de 0° C à 25 ° C.

**9.** Procédé selon la revendication 2, dans lequel la réaction mise en évidence dans la voie de synthèse 1 est effectuée avec un rapport molaire entre les composés de la formule III et les amines secondaires de la formule IV de 1 : 1,5 et 1 : 4.

**10.** Procédé selon les revendications 2 et 9, dans lequel les amines secondaires de la formule IV dont les $R_1$ et $R_2$ sont

des chaînes d'alkyle ou d'alcényle linéaire ou ramifié, sont de préférence de 1 à 30 atomes de carbone ; ou des groupes du cycle alkyle ou aryle.

**11.** Procédé selon les revendications 2 et 9, dans lequel la réaction mise en évidence dans la voie de synthèse 1 pour la préparation des amines tertiaires de la formule V, est effectuée en présence de solvants tels que l'acétonitrile, le chloroforme, le diméthylformamide, le diméthylsulfoxyde, l'acétone ou les alcools à chaîne courte.

**12.** Procédé selon les revendications 2 et 9, dans lequel la réaction mise en évidence dans la voie de synthèse 1 pour la préparation des amines tertiaires de la formule V, est effectuée dans un temps de réaction de 5 à 60 heures.

**13.** Procédé selon les revendications 2 et 9, dans lequel la réaction mise en évidence dans la voie de synthèse 1 pour la préparation des amines tertiaires de la formule V, est effectuée à une température de 50 °C à 150 °C.

**14.** Procédé selon les revendication 2, dans lequel la réaction mise en évidence dans la voie de synthèse 1 parmi les composés de la formule V avec le sodium-2-bromoéthanesulfonate ou le sodium-3 -chloro-2-hydroxypropanesulfonate de la formule VI est effectuée avec un rapport molaire de 1 : 1,5 à 1 : 1,4.

**15.** Procédé selon les revendications 2 et 14, dans lequel la réaction mise en évidence sur la voie de synthèse 1 pour la préparation des composés de la formule VII est effectuée en présence de solvants tels que l'eau, des alcools et des hydrocarbures inertes.

**16.** Procédé selon les revendications 2 et 14, dans lequel le temps de réaction, la température et la pression pour la préparation des composés de la formule VII mise en évidence sur la voie de synthèse 1, dépendent de la structure des composés des formules V et VI.

**17.** Procédé selon la revendication 16, dans lequel le temps de réaction varie de 6 à 72 heures.

**18.** Procédé selon la revendication 16, dans lequel la température est de 40 à 180 °C.

**19.** Procédé selon la revendication 16, dans lequel la pression varie de 779 à 1013 hPa (585 à 760 mmHg) et est de préférence atmosphérique.

**20.** Procédé selon la revendication 20, dans lequel les polyéthylènes glycols de la formule 1 utilisée dans la voie de synthèse 2 contiennent deux groupes époxyde, l'un à l'extrémité et l'autre au début de la chaîne polymère.

**21.** Procédé selon la revendication 2, dans lequel le poids moléculaire des polyéthylène glycols de la formule 1 utilisée dans la voie de synthèse 2 est sélectionné dans la plage de 100 à 22.000 g/mol.

**22.** Procédé selon la revendication 2, dans lequel la réaction décrite dans la voie de synthèse 2 entre les composés de la formule 1 et les amines primaires ou secondaires de la formule II est effectuée avec un rapport molaire de 1 : 1,5 à 1 : 4.

**23.** Procédé selon les revendications 2 et 4, dans lequel dans les amines primaires et secondaires utilisées dans la voie de synthèse 2 de la formule IV, dont $R_1$ et $R_2$ sont des chaînes alkyle et alcényle linéaires ou ramifiées, portant de préférence 1 à 30 atomes de carbone ; ou des groupes du cycle alkyle ou aryle.

**24.** Procédé selon les revendications 2 et 4, dans lequel la réaction mise en évidence sur la voie de synthèse 2 pour la préparation des amino-alcools de la formule V, est effectuée en présence de solvants tels que l'acétonitrile, le dioxane, le chloroforme, la diméthylformamide, le diméthylsulfoxyde, l'acétone ou les alcools à chaîne courte.

**25.** Procédé selon les revendications 2 et 4, dans lequel la réaction décrite dans la voie de synthèse 2 pour la préparation des amino-alcools de la formule V, est effectuée dans un temps de réaction de 6 à 48 heures, de préférence de 12 à 20 heures.

**26.** Procédé selon les revendications 2 et 4, dans lequel la réaction décrite dans la voie de synthèse 2 pour la préparation des amino-alcools de la formula V, est effectuée à une température de 50 °C à 150 °C, de préférence de 60 à 90 °C.

**27.** Procédé selon la revendication 2, dans lequel la réaction décrite dans la voie de synthèse 2 entre les composés de

la formule V avec du sodium-2-bromoéthanesulfonate ou du sodium 3-chloro-2-hydroxypropanesulfonate de la formule VI, est effectuée avec un rapport molaire de 1 : 1,5 à 1,4.

**28.** Procédé selon les revendications 2 et 27, dans lequel la réaction décrite dans la voie de synthèse 2 pour obtenir les composés de la formule VII est effectuée en présence de solvants tels que l'eau, le dioxane, des alcools, des aromates ou des hydrocarbures.

**29.** Procédé selon la revendication 27, dans lequel le temps de réaction varie de 6 à 48 heures.

**30.** Procédé selon la revendication 27, dans lequel la température est de 40 à 180 °C.

**31.** Procédé selon la revendication 27, dans lequel la pression varie de 779 à 1013 hPa (585 à 760 mmHg) et est de préférence atmosphérique.

**32.** Utilisation de liquides zwittérioniques géminaux ramifiés, à base de sulfobétaïne et d'hydroxysultaïne de la formule structurale décrite dans la revendication 1 dans des procédés de récupération améliorée du pétrole, dans lesquels la mouillabilité des roches telles que le calcaire, la dolomite, le grès, le quartz ou les lithologies hétérogènes est modifiée.

**33.** Utilisation de liquides zwittérioniques géminaux ramifiés selon la revendication 32 dans des procédés de récupération améliorée du pétrole, dans lesquels il y a la présence de saumures présentant une teneur élevée en sels et en ions divalents tels que le calcium, le magnésium, le baryum et le strontium.

**34.** Utilisation de liquides zwittérioniques géminaux selon la revendication 32, dans lesquels la température peut atteindre 220 °C.

**35.** Utilisation de liquides zwittérioniques géminaux selon la revendication 32, dans lesquels la pression peut atteindre 5.516*107 Pa (8000 psi).

**36.** Utilisation de liquides zwittérioniques géminaux selon la revendication 32, dans lesquels la concentration en sels peut atteindre 400.000 ppm.

**37.** Utilisation de liquides zwittérioniques géminaux selon la revendication 32, dans lesquels la concentration en ions divalents peut atteindre 180.000 ppm.

**38.** Utilisation de liquides zwittérioniques géminaux selon la revendication 32, dans lesquels la concentration du liquide zwitterionique à injecter afin de modifier la mouillabilité de la roche pétrolière se situe à des concentrations de 25 à 40.000 ppm.

**39.** Utilisation de liquides zwittérioniques géminaux selon la revendication 38, dans lesquels la concentration à injecter est de 500 et 10.000 ppm.

**40.** Utilisation de liquides zwittérioniques géminaux de la formule structurale selon la revendication 1, en tant qu'inhibiteurs de la corrosion de métaux ferreux utilisés dans des conduites et des équipements utilisés pour des opérations d'extraction et de transport de pétrole brut.

**41.** Utilisation de liquides zwittérioniques géminaux selon la revendication 40, dans lesquels la concentration du liquide zwittérionique à injecter afin d'inhiber la corrosion est de 25 à 500 ppm.

**42.** Utilisation de liquides zwittérioniques géminaux selon la revendication 41, dans lesquels la concentration à injecter est sélectionnée pour être de 50 à 300 ppm.

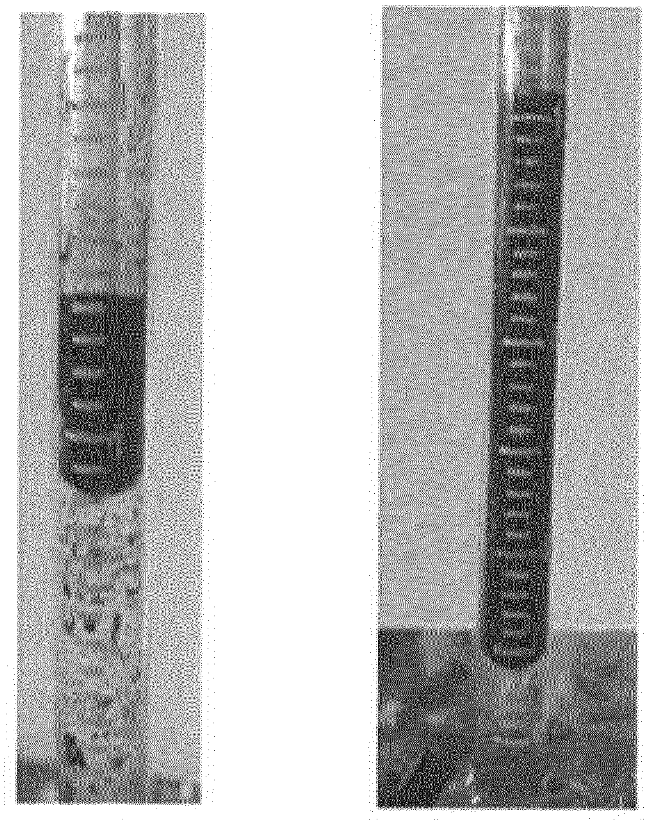

a) b)

Figure 1

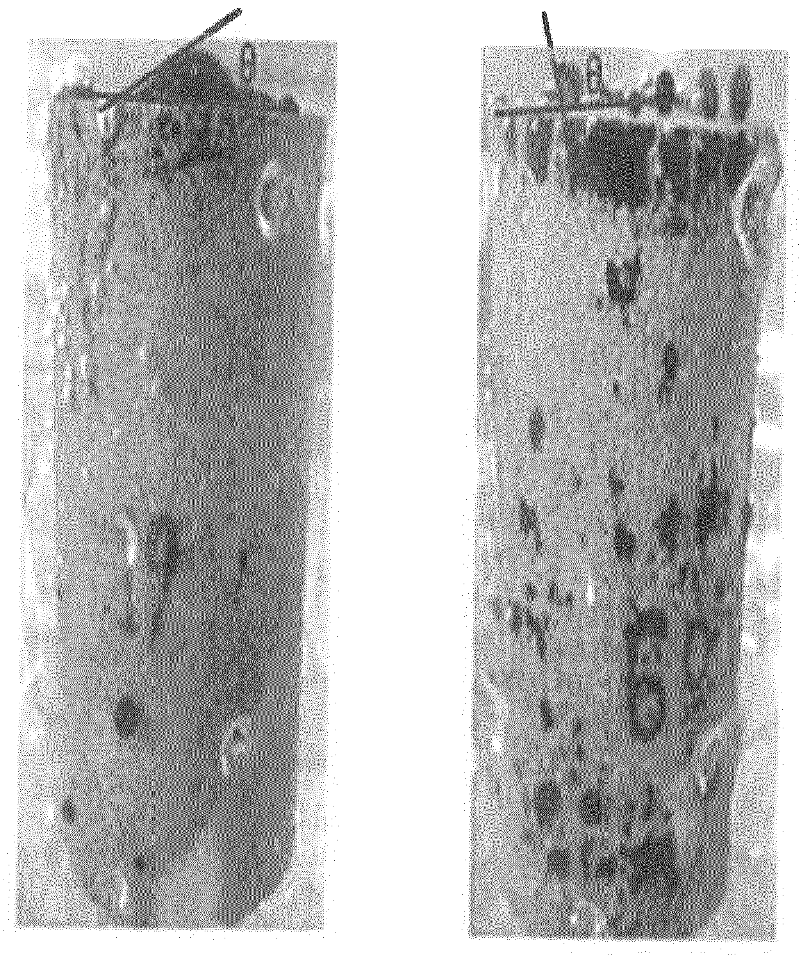


a)    b)

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5042580 A
- US 4509951 A
- US 20090023618 A1
- US 4842065 A
- US 3643738 A
- US 2011138683 A
- US 4900458 A
- US 4275744 A
- US 5084210 A
- US 4039336 A
- US 6521028 B
- US 20060013798 A
- US 8105987 B
- US 20110138683 A


### Non-patent literature cited in the description


- *Chemistry Letters,* 2004, vol. 33 (12), 1594-1595
- *Latvian Journal of Chemistry,* 2010, (1-4), 102-113
- *Monatshefte für Chemie,* 2008, vol. 139, 799-803
- *Journal of Chemical Society of Mexico,* 2002, vol. 46 (4), 335-340
- *Applied Surface Science,* 2006, vol. 252 (6), 2139-2152
- *Colloids Surf. A: Physicochem. Eng. Aspects,* 1998, vol. 137, 117-129
- **AUSTAD, T. ; MATRE, B. ; MILTER, J. ; SAEVAREID, A. ; OYNO, L.** Chemical flooding of oil reservoirs 8. Spontaneous oil expulsion from oil- and water-wet low permeable chalk material by imbibition of aqueous surfactant solutions. *J. Pet. Sci. Eng.,* 2000, vol. 28, 123-143